# EUROPEAN PATENT SPECIFICATION

(11) **EP 3 740 199 B1**
(45) Date of publication and mention of the grant of the patent: **14.08.2024**
(21) Application number: 19743691.8
(22) Date of filing: 17.01.2019
(51) Int. Cl.: A61K 9/51, C12N 15/86

(54) **POLYMER-ENCAPSULATED VIRAL VECTORS FOR GENETIC THERAPY**
POLYMERVERKAPSELTE VIRALE VEKTOREN FÜR DIE GENTHERAPIE
VECTEURS VIRAUX ENCAPSULÉS DANS UN POLYMÈRE DESTINÉS À LA THÉRAPIE GÉNIQUE

(30) Priority: 17.01.2018 US 201862618156 P; 24.04.2018 US 201862661990 P; 06.08.2018 US 201862715007 P
(43) Date of publication of application: 25.11.2020
(73) Proprietor: IXAKA FRANCE, 94800 Villejuif (FR)
(72) Inventor: BAUCHE, Cecile, 75013 Paris (FR); VAILLANT, Renaud, 94250 Gentilly (FR); SARRY, Emeline, 92240 Malakoff (FR); MOURLANE, Frederic, 06000 Nice (FR); BISHOP, Philippe, Foster City, CA 94404 (US)
(74) Representative: Cabinet Beau de Loménie
(86) International application number: PCT/IB2019/000361
(87) International publication number: WO 2019/145796

(56) References cited:
- EP-A1- 3 406 265
- WO-A1-2014/093966
- WO-A1-2014/136100

## Description

### BACKGROUND

Gene therapy delivers exogenous genetic material to the target cells in order to correct genetic abnormalities or provide treatment of a disease by altering cell function. To this end, both viral and non-viral gene delivery methods have been used, but both approaches still present significant shortcomings.

A number of viral vector applications have already been translated into the clinic, either for gene therapy or vaccination protocols. Nevertheless, currently used viral vectors have certain disadvantages. For example, it is challenging to target specific cells using a viral vector. In some gene therapy protocols, cell targeting is achieved by purifying the target cells and transducing them *ex vivo* and expanding the transduced cells *in vitro* before re-implanting them into the patient. In vaccination protocols, direct injection of the vector is performed, and nonspecific cell transduction is often used to elicit an immune response against the gene product encoded by the vector. To increase efficacy and safety of the treatment, cell targeting could be required.

There is a need for improved viral vector-based systems for targeted delivery of genetic material.

### SUMMARY

The technology described herein provides synthetic packaged viral vector nanoparticle compositions and methods for using them to provide enhanced delivery of genetic material for use in gene therapy and vaccine applications. The vector compositions and methods can be employed in any situation for which transduction of cells with one or more transgenes is useful. For example, they can be used for treatment of cancer, infectious diseases, metabolic diseases, neurological diseases, or inflammatory conditions, or to correct genetic defects.

The viral vector nanoparticle compositions of the present technology include an outer shell containing a polymer or a mixture of polymers which encapsulate the vector.

In a first aspect, the present invention relates to a nanoparticle comprising a viral vector coated with a polymer or a mixture of polymers, wherein the viral vector comprises a transgene, and wherein the nanoparticle functions as a vehicle for delivering the transgene to eukaryotic cells, wherein the viral vector lacks envelope protein

In certain embodiments, the polymer or the mixture of polymers comprises a poly(beta-amino ester) having the general formula 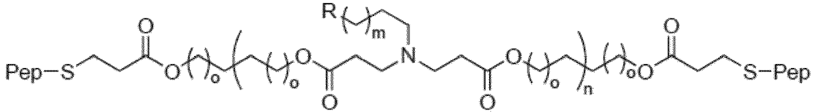 wherein each Pep is an oligopeptide, wherein R is OH, CH₃, or a cholesterol, and wherein m ranges from 1 to 20, n ranges from 1 to 100, and o ranges from 1 to 10.

In preferred embodiments, the oligopeptide includes at least three amino acid residues, selected from the group consisting of arginine (R), lysine (K), histidine (H), glutamic acid (E), aspartic acid (D), and cysteine (C). See also WO2014/136100 and WO2016/116887 for further description of oligopeptide-derivatized PBAEs which can be used in the present technology. Cysteine can be included to provide a covalent attachment point for the peptide to the polymer; while it carries a slight negative charge at pH 7, it does not significantly alter charge if used together with positively charged amino acids. Exemplary peptides are CRRR (SEQ ID NO:1), CHHH (SEQ ID NO:2), CKKK (SEQ ID NO:3), CEEE (SEQ ID NO:4), and CDDD (SEQ ID NO:5). In other embodiments, any naturally occurring amino acid can be included in the Pep moieties. The sequence of the peptide is selected so as to promote cellular uptake and targeting of the polymer-coated vector. In embodiments both utilizing and lacking viral envelope proteins, the oligopeptide sequences and net charge are selected so as to promote cellular uptake and/or endosomal uptake and/or endosomal escape of viral vectors encapsulated with the polymers in the intended target cells. Peptides at each of the two ends of the polymer typically are the same on a given polymer molecule, but may be different. Polymer molecules in a mixture used to coat a vector may be the same or different; if different, they may differ in the polymer backbone or in the terminal peptides. In preferred embodiments, the peptide at both ends of the polymer has the amino acid sequence CRRR (SEQ ID NO:1), or CHHH (SEQ ID NO:2), or CKKK (SEQ ID NO:3), or CEEE (SEQ ID NO:4), or CDDD (SEQ ID NO:5). The polymer or mixture of polymers can have a net positive or negative charge, or can be uncharged. Oligopeptides containing 2 or more residues of only a single type of amino acid, such as R, K, H, D, or E, can be used. The polymer can comprise a sugar or sugar alcohol grafted onto the lateral chain attached to the polymer backbone. One or more of the polymer molecules encapsulating each vector nanoparticle can optionally comprise a targeting moiety linked to the polymer at any desired position on the polymer molecule, such as at R. The polymer molecules can be cross-linked or non-cross-linked. The polymer molecules can be attached to the viral vector surface either non-covalently, or covalently such as by covalent attachment to a lipid molecule (e.g., cholesterol, a phospholipid, or a fatty acid) that partitions into the lipid bilayer of the vector, or to a protein embedded in the lipid bilayer. In some embodiments, the polymer merely coats the vector but is attached non-specifically, i.e., it is not attached to the vector by specific covalent or non-covalent interactions, but only by nonspecific interactions such as net charge, hydrophobicity, or van der Waals interactions. In certain embodiments, the ratio of the polymer or mixture of polymers to the retroviral vector is from about 1:100 to about 5:1 by weight, or the number of polymer molecules per vector particle is from about 10⁶ to about 10¹².

The viral vector nanoparticles comprise a viral vector. The viral vector can be chosen based on desired characteristics (e.g., immunogenicity, capacity to accommodate different sized constructs, integrating and replicative properties, expression level, duration of expression, tissue tropism or targeting characteristics, ability to infect dividing and/or quiescent cells, etc.), The viral vector can be a retroviral vector such as a lentiviral vector. In preferred embodiments, the viral vector is a retroviral vector. However, the invention can also be practiced with other types of virus and/or viral vectors, including those derived from DNA or RNA viruses.

The nanoparticles can include one or more targeting moieties that are exposed at the surface of the encapsulated vector nanoparticles, such as by covalent or non-covalent association of the targeting moieties with the polymer coating. One molecular species of targeting moiety, or more than one different molecular species of targeting moiety, can be present on each viral vector nanoparticle. Targeting moieties can be, for example, antibodies, antibody fragments (including Fab), scFvs, antibody-like protein scaffolds, oligopeptides, aptamers, L-RNA aptamers, or ligands for cell surface receptors. In an embodiment, the targeting moiety is an anti-CD3 antibody or aptamer for targeting the nanoparticles towards T-cells. In an embodiment, the transgene encodes a chimeric antigen receptor. The nanoparticle is capable of acting as a gene delivery vehicle by transducing cells *in vivo* in a subject to whom the vehicle is administered, or transducing cells *in vitro.* In certain embodiments, the nanoparticle is capable of transducing a specific type of cell or class of cells, usually through the action of the targeting moiety and/or through the action of the polymer or polymer mixture. In some embodiments, the polymer of polymer-coated vector particles can promote cell transduction, not only by attachment to target cell surfaces, but through endosomal uptake, such as by endocytosis, micropinocytosis, phagocytosis, or other mechanisms, and endosomal escape (via membrane fusion after a reduction in pH in the endosomal vesicle). In preferred embodiments, the amino acid sequence of oligopeptides associated with the polymer can specifically promote cell transduction via the endosomal route.

The vectors of the invention lack envelope proteins; which differs from pseudotyped vectors. In pseudotyped vectors, envelope proteins such as the HIV gp120-gp41 complex or the vesicular stomatitis virus (VSV-G) glycoprotein form spike-like structures on the outer surface of the viral envelope, which facilitate attachment of the vector particles to host cells and entry of viruses into host cells. Pseudotyping proteins such as VSV-G also can be used to protect or bind the vector during purification (e.g., protection during ultracentrifugation, binding to an affinity column or other affinity matrix, or gel purification). However, in the context of polymer packaging of a viral vector, such structures can interfere with tight association between the vector particle envelope and the polymers. Viral envelope proteins also carry a pH-dependent charge, which can limit or interfere with the association of polymers, particularly charged polymers. Further, packaged viral vectors harboring pseudotyping proteins can undergo coating destabilization and destruction in vitro and in vivo, thus releasing viral vector able to transduce cells unspecifically, potentially leading to a reduction in safety profile.

Unlike pseudotyped vectors, vectors lacking envelope proteins can enhance the packaging of viral vectors using polymers. Coated vectors lacking envelope protein show an improved safety profile compared to vectors having envelope protein, because after destabilization or destruction of the coating, they are not able to transduce cells in vitro or in vivo. The nanoparticles of the present technology wherein the viral vector lacks viral envelope protein have a built-in safety mechanism for use in gene therapy or immunotherapy, because the nanoparticles are only capable of transducing a mammalian cell above a threshold number of polymer molecules per vector. Below that threshold number of polymer molecules per vector, the amount of polymer is insufficient to completely coat the vector, which can cause the nanoparticles to become structurally unstable or subject to dissociation of polymer molecules from the nanoparticle. Once such vectors lacking envelope protein lose an effective polymer coating, they become incapable of transducing mammalian cells, including human cells. Such nanoparticles have an improved safety profile for in vivo use compared to a vector or polymer-encapsulated vector that lacks the threshold feature.

In some embodiments, some membrane proteins, such as proteins that are not viral envelope or fusion proteins and do not otherwise form spike-like structures on the membrane outer surface, such as proteins from the vector producing cells not used for pseudotyping, may be present in the lipid membrane of the viral vector particle. In certain embodiments of the present technology, viral envelope proteins are excluded from the viral vector particles, such as those which have a significant mass protruding from the outer surface of the envelope lipid bilayer, such as at least 20%, at least 30%, at least 40%, or at least 50% of their mass protruding from the outer envelope surface.

The nanoparticle compositions optionally can contain additional components, such as lipid molecules, surfactants, nucleic acids, protein molecules, or small molecule drugs. The present technology also contemplates pharmaceutical formulations or compositions containing the nanoparticles together with one or more excipients, carriers, buffers, salts, or liquids, rendering the delivery vehicle suitable for administration via oral, intranasal, or parenteral administration, such as intravenous, intramuscular, subcutaneous, peritumoral or intratumoral injection, or for *in vitro* administration to cells in an *ex vivo* gene transfer protocol. Such compositions and formulations can also be lyophilized to stabilize them during storage.

The viral vector nanoparticle of the present technology can serve as a gene delivery vehicle. The nanoparticle includes a viral vector coated on its exterior surface with a layer containing a polymer or a mixture of polymers. In certain embodiments, the retroviral vector specifically lacks the viral envelope protein that might typically be included in the envelope of similar retroviral vectors. In certain embodiments, the viral vector specifically lacks any naturally occurring or modified viral vector envelope proteins, such as wild type or modified VSV-G, HIV gp120, HIV gp41, MMTV gp52, MMTV gp36, MLV gp71, syncytin, wild type or modified Sindbis virus envelope protein, measles virus hemagglutinin (H) and fusion (F) glycoproteins, and HEMO. In other embodiments, the vector contains one or more of such envelope proteins.

In a second aspect, the present invention provides a method of making the above described nanoparticle/gene delivery vehicle (viral vector nanoparticle). The method includes the steps of: (a) providing a viral vector lacking envelope protein and containing a transgene; (b) providing a polymer or a mixture of polymers; and (c) contacting the viral vector and the polymer or mixture of polymers, whereby the viral vector and the polymer or the mixture of polymers combine to form the nanoparticle, which contains the viral vector coated with the polymer or mixture of polymers.

In a third aspect, the present invention provides the nanoparticle according to the first aspect of the present invention for use in a method of treating a disease, the method comprising administering a composition comprising a plurality of said nanoparticles to a subject in need thereof, whereby cells of the subject are transduced by the retroviral vector and the transgene is expressed in the transduced cells. In one embodiment, the disease to be treated is cancer. For in vivo administration, embodiments in which viral envelope proteins such as VSV-G are absent are preferred, as they provide an enhanced safety profile because they lack the ability to transduce non-targeted cells in the host and due to more specific targeting provided by the use of polymer encapsulation to restore cellular uptake and/or endosomal uptake and/or endosomal escape otherwise provided by envelope protein.

In a fourth aspect, the present invention provides a method of transducing cells *in vitro.* The method includes contacting cultured cells with a plurality of nanoparticles according to the first aspect of the invention, whereby at least some of the cultured cells are transduced by the retroviral vector and the transgene is specifically expressed in the transduced cells. In some embodiments, the nanoparticles are targeted to CD3-positive cells and the transgene is specifically expressed in CD3-positive cells, or the transgene encodes a chimeric antigen receptor.

In a fifth aspect, the present invention provides the nanoparticle of the first aspect of the present invention for use in a method of performing gene therapy, the method comprising contacting cells in vitro or within a living organism with a plurality of said nanoparticles, whereby the transgene is expressed in the cells. The transgene can replace or modify a deficient gene or replace a missing gene.

The present invention technology is further described in the claims.

### BRIEF DESCRIPTION OF THE DRAWINGS

Figure 1A shows a schematic diagram of an embodiment of a nanoparticle for gene delivery. The nanoparticle contains a polymer-encapsulated viral vector particle which lacks envelope protein. Figure 1B shows a schematic diagram of a nanoparticle not according to the invention for gene delivery, wherein the viral vector has envelope proteins.
Figure 2 shows a schematic diagram of an embodiment of a nanoparticle for gene delivery, wherein the viral vector is encapsulated in multiple polymer layers. Viral vector envelope proteins, while not depicted, may be present in some embodiments.
Figure 3 shows a schematic diagram of an embodiment of a nanoparticle for gene delivery, wherein the nanoparticle includes a targeting moiety attached to the polymer layer (or outer polymer layer if a polymer multilayer is used).
Figures 4A-4C are schematic representations of a process of transducing a cell with a lentiviral vector containing a GFP transgene (FIG. 4A), inhibiting the transduction using an anti-VSV-G antibody (FIG. 4B), and preventing the antibody mediated inhibition by polymer encapsulation of the vector particles (FIG. 4C).
Figures 5A-5C are schematic representations of a process similar to that depicted in FIGS. 4A-4C, but using a lentiviral vector lacking VSV-G protein.
Figures 6A-6F show results of zeta potential measurements on bald lentiviral vectors encapsulated with the indicated PBAE polymers.
Figure 7 shows results of transduction of HEK293T cells with lentiviral vector containing a GFP transgene as a function of incubation time in formulation buffers varying in composition and pH.
Figures 8A-8H show the physical stability of a lentiviral vector lacking VSV-G protein as a function of free p24 released after increasing incubation times (hours) in the indicated formulation buffers varying in composition and pH. Figs. 8A-8D show LV associated p24, and Figs. 8E-8H show free p24 released from the LV.
Figure 9 shows results of transduction of HEK293T cells with lentiviral vector lacking VSV-G protein and containing a GFP transgene as a function of R polymer/vector particle ratio, formulation buffer composition and pH.
Figure 10 shows results of an experiment similar to that shown in Fig. 9 but using R/H polymer mix in which both polymers were or were not conjugated with cholesterol.
Figure 11 shows results of an experiment similar to that shown in Fig. 9 but using a pseudotyped lentiviral vector containing a GFP transgene.
Figure 12 shows results of GFP expression and cell viability after transduction using VSV-G+ lentivectors encapsulated using the indicated PBAE polymers and polymer molecule/cell ratios, with and without VSV-G antibody. The data are normalized, with the transduction in the absence of the antibody equaling 100%.
Figures 13A - 13D show results of mCherry expression and cell viability after transduction using VSV-G⁻ (bald) lentivectors encapsulated using the indicated PBAE polymers and polymer molecule/cell ratios.

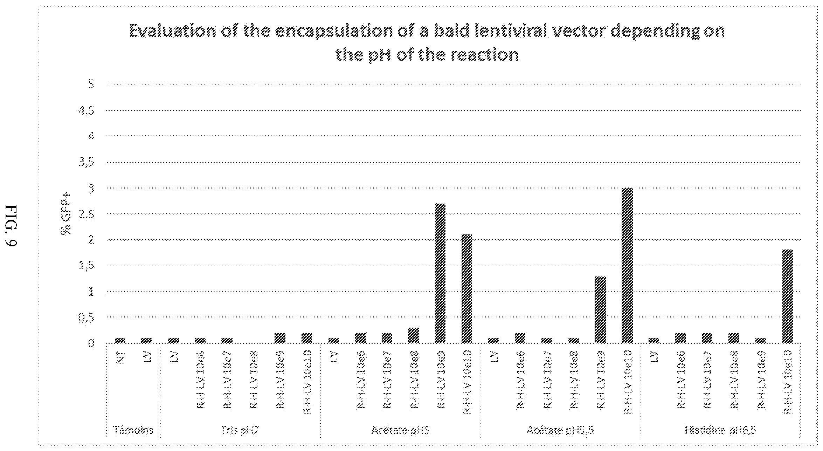

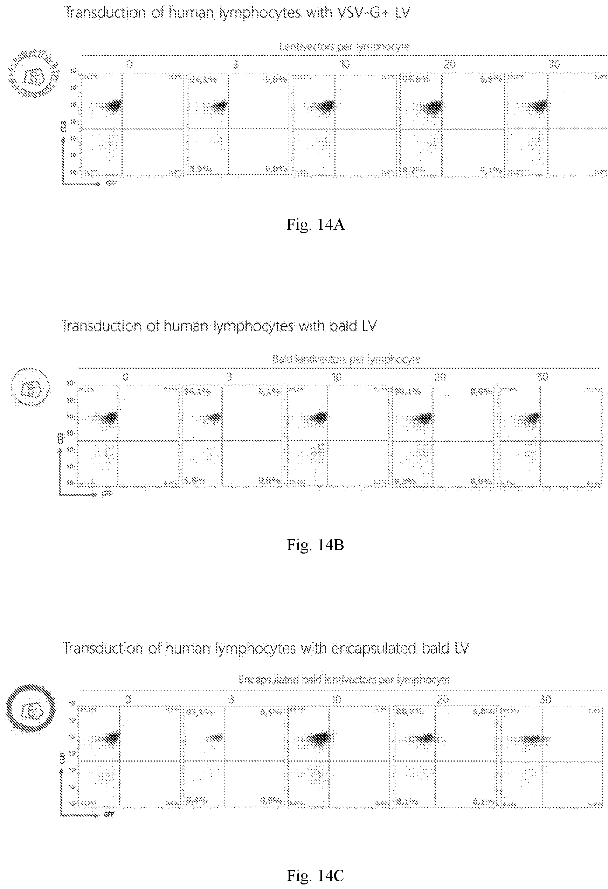

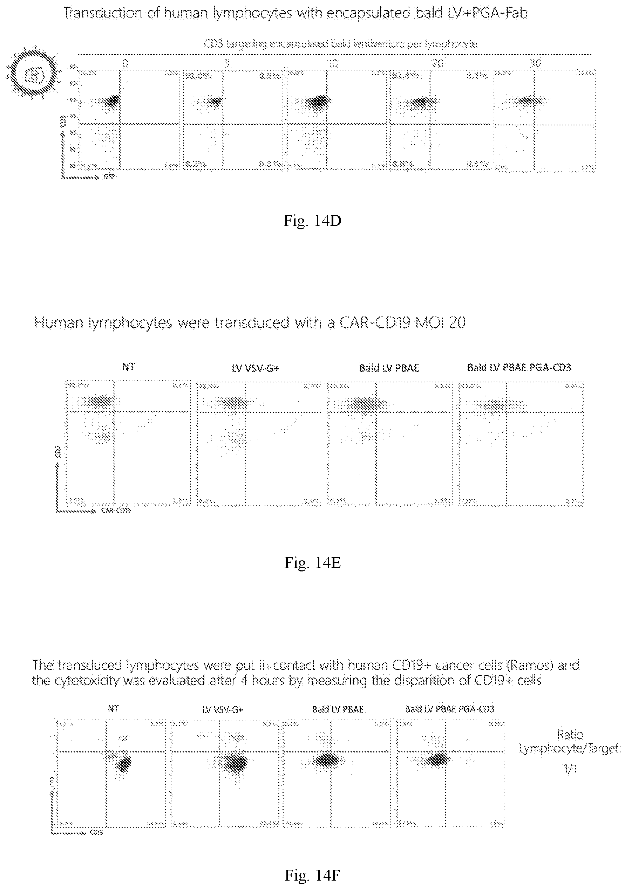

Figures 14A - 14F show results of transduction of human lymphocytes nonencapsulated VSV-G⁺ lentiviral vectors (14A), nonencapsulated bald LV (14B), PBAE-encapsulated bald LV (14C), and PBAE-encapsulated bald LV with model targeting moiety PGA-anti-CD3 Fab (14D), all analyzed by fluorescence activated cell sorting (comparing CD3 antibody labeling and GFP expression). In Fig. 14E, the lymphocytes also had been transduced with a CAR specific for CD19, and cells expressing CD3 are plotted against the CD19 CAR expression. In Fig. 14F the same lymphocytes as used for the experiment depicted in Fig. 9E were contacted with human CD19+ cancer cells (Ramos cells) and cytotoxicity against the cancer cells evaluated by loss of CD19+ cells after 4 hours.
Figure 15 shows results of transduction (GFP expression) in different populations of mouse PBMCs after treatment with each of the indicated vectors and titers.

### DETAILED DESCRIPTION

Packaged viral vector nanoparticle compositions that allow enhanced delivery of genetic material for gene therapy and vaccine applications are provided. These compositions include a viral vector encapsulated by polymer or a mixture of polymers. The polymer shell provides protection of the viral vector particle in the bloodstream and tissues following parenteral administration to a subject and in some embodiments can assist in delivering the vector to its target cell.

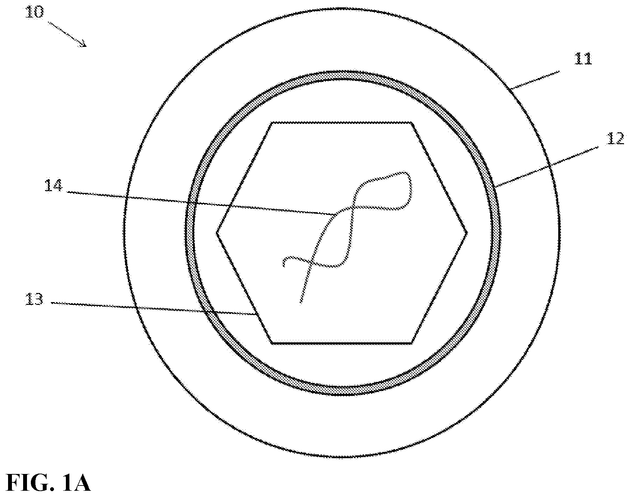

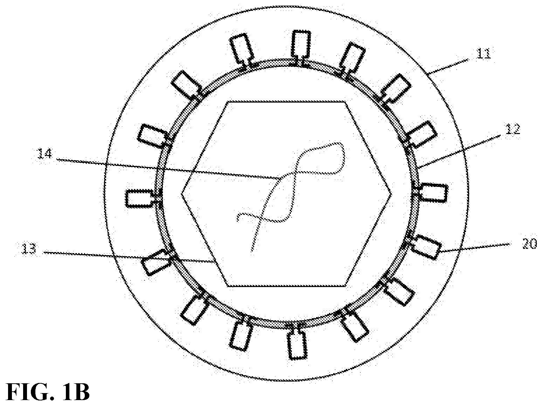

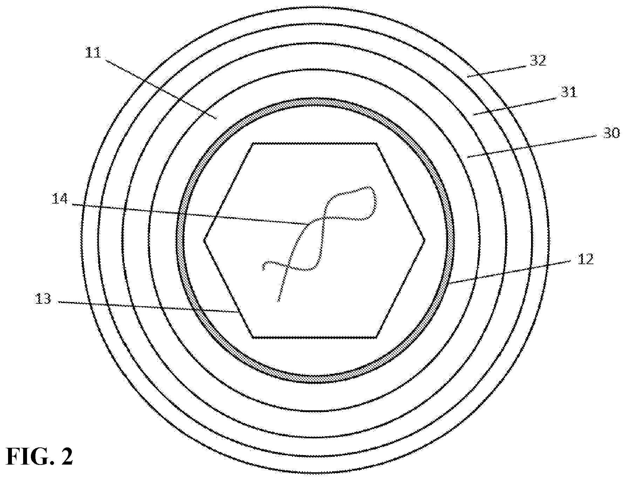

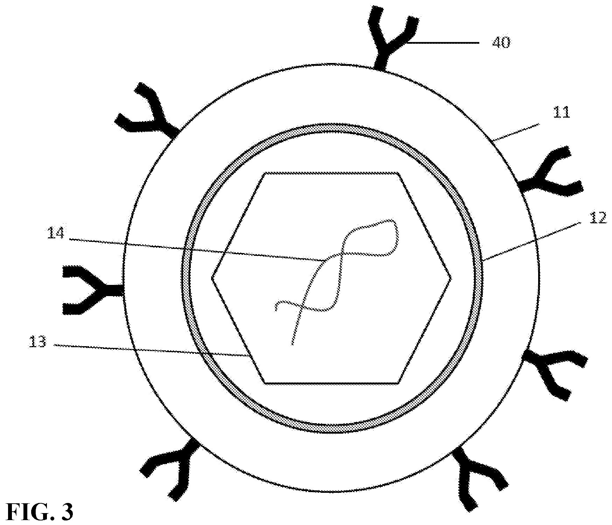

Fig. 1A shows a viral vector nanoparticle, also referred to herein as gene delivery vehicle, according to the present technology. Fig. 1B shows a viral vector nanoparticle, also referred to herein as gene delivery vehicle, not according to the present technology. The basic structure of the vehicle is that of nanoparticle 10, which contains a retroviral vector encapsulated by polymer shell 11. The retroviral vector includes membrane envelope 12, protein capsid 13 disposed within the envelope, and one or more nucleic acid molecules 14 disposed within the capsid. The viral envelope is a lipid bilayer-based structure that, in the viral vector nanoparticle shown in Fig. 1B, contains one or more types of viral envelope or fusion proteins 20 embedded within or attached to the bilayer membrane; such envelope or fusion proteins are lacking in the viral vector nanoparticle depicted in Fig. 1A. The viral vector envelope, with or without envelope proteins, is encapsulated, both functionally and essentially entirely, by polymeric matrix 11. The vector particle can be encapsulated by more than one polymer shell (see, e.g., Fig. 2), i.e., can be encapsulated by a multilayered polymer shell. Figure 3 shows an embodiment in which targeting moiety 40 is associated with the polymer shell.

In some embodiments, the present technology provides a novel generation of synthetic viral vectors, particularly lentiviral vectors, in which the viral vector particles lack a viral envelope protein. This new platform technology confers several advantages compared to using viral vector particles containing an envelope protein, including cheaper production of viral vector particles by transient transfection. 1) Due to removal of the plasmid coding for the viral envelope protein and due to the fact that the vector is not transducing the producing cells during production, deleting the envelope protein increases the useful production time so the yields of produced vectors. 2) Vectors lacking envelope protein are safer for use for in *in vivo* gene therapy, because the uncoated vectors, lacking a viral envelope protein, cannot transduce cells, and because they have reduced immunogenicity, 3) Vectors lacking envelope protein provide greater accuracy and efficiency of transduction due to the use of specific polymer-mediated targeting. 4) Polymer-encapsulated viral vectors, particularly lentiviral vectors, are more stable at low pH than ordinary (uncoated) viral vectors, which improves their stability and effectiveness in use, especially when endosomal uptake mechanisms are involved. Elimination of viral envelope protein from viral vector particles is especially compatible with the use of polymer-coated viral vector particles, because it essentially eliminates the ability of the uncoated vector particles to transduce cells, and the polymer coating can provide specific targeting; thus, the risk of transducing non-targeted cells is drastically reduced. Further, the elimination of viral envelope protein promotes the tight association of polymer molecules with the surface of the vector envelope, providing better stability and more reliable targeting compared to polymer coated viral vector particles containing viral envelope proteins.

In order to produce the nanoparticulate form of the vector, polymer molecules must be tightly adhered to the vector particles. Loose association of the polymer molecules with the vector can lead to delamination and loss of the polymer shell from the surface of the viral vector particles, with subsequent loss of activity. Viral vectors based on retroviruses typically possess a core structure or capsid, which contains a transgene and other necessary nucleic acid sequences, and the core structure is in turn surrounded by a membrane envelope. The envelope typically contains a viral fusion protein that assists in attachment and fusion of the viral envelope with the host cell membrane, allowing cellular entry of the viral vector. However, in the embodiment of the present technology, the role of cell targeting, attachment, and entry is assumed (and restored in the case of bald LV) by the polymer shell of the nanoparticulate vector particles, which can act at the cellular uptake and/or endosomal level to transduce cells, rendering the viral fusion protein unnecessary. Further, the presence in the vector envelope of a viral fusion protein interferes with the tight and stable attachment of polymer molecules to the envelope. Therefore, in certain embodiments of the present technology, the viral vector nanoparticles have been engineered to lack envelope proteins, particularly to lack viral fusion or "spike" proteins. The result is a more robust nanoparticle structure and a more stable vector that exhibits better activity and longer half-life when administered to a patient, such as by intravenous administration.

### Poly(beta-amino ester) nanoparticles

In the present technology, for a viral vector to "lack envelope protein" means that the envelope, in particular the lipid bilayer, of the vector envelop is devoid of any proteins typically found on the surface of enveloped viruses, in either their naturally occurring or modified form, such as the Env protein complex or viral envelope glycoproteins exemplified by HIV gp120-gp41 complex, vesicular stomatitis virus glycoprotein (VSV-G), influenza virus hemagglutinin, measles virus H and F proteins, and wild type or modified Sindbis virus envelope protein. Such proteins, which the vector nanoparticles of the present technology lack, would, if present, project outward from the lipid bilayer of the vector envelope and interfere with the stable contact required between the outer polymer layer and the lipid bilayer. In different embodiments, viral vector nanoparticles of the present technology can either i) lack envelope proteins entirely, ii) essentially lack envelope proteins (i.e., contain only a trace amount such as 0.1% or less of typical viral vector particles), or iii) have a small amount of envelope protein, such as less than 10%, less than 5%, less than 3%, less than 2%, or less than 1% of the typical amount. A typical amount of envelope protein is an amount present in a viral vector particle that relies on the envelope protein for cellular entry and transduction, or an amount found in the envelope of an intact, functional virus particle. The envelope of the viral vector can be engineered to completely lack envelope protein, for example by eliminating any gene encoding an envelope protein during expression in the cells from which the vector is produced. Membrane-embedded or membrane attached proteins that do not form spike-like structures on the outer surface of the envelope may, however, be present in the lipid bilayer of the viral vector nanoparticles in certain embodiments.

In one embodiment, the polymers used to form the viral vector nanoparticle include one or more species of poly(beta-amino ester) (PBAE) having the formula 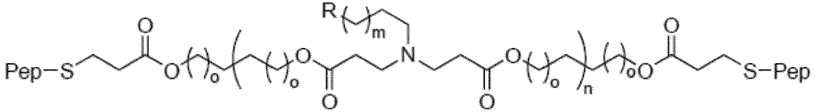 wherein Pep is an oligopeptide and R is OH, CH₃, or cholesterol, a phospholipid molecule, or a fatty acid or acyl chain, wherein m preferably ranges from 1 to 20. n preferably ranges from 1 to 100, and o ranges from 1 to 10. Other polymers and mixtures thereof with one another and with PBAE also can be used to encapsulate viral vector particles. Further coating mechanisms using lipids, such as in the form of unilamellar or multilamellar lipid liposomes or micelle-like structures including surfactants and/or lipid molecules, can be used as well. Such coatings can be especially useful for encapsulating viral vector particles lacking envelope proteins ("bald" vectors).

PBAEs have been described as non-viral polynucleotide delivery vectors capable of condensing both DNA and RNA into discrete nanoparticles (Green, J.J. et al. Acc. Chem. Res. 41, 749-759 (2008)), though they show low transduction efficiency and high toxicity. PBAEs can be formed from the reaction of di(acrylate ester) monomers with amine monomers, resulting in polymers having terminal acrylate groups that can be functionalized with end modifications. End groups are functionalities or constitutional units that are at an extremity of a macromolecule or oligomer. End modification of PBAEs allows for the improvement of their biological properties.

Chemical modification at the termini of PBAEs with primary amines, for example, produce higher transfection efficacy than acrylate-terminated polymers. Alternatively, PBAEs can be end modified with oligopeptides, which can target the vector to particular cell types, depending on the specific oligopeptide sequence. An "oligopeptide" as defined here comprises a string of at least two, or in some embodiments at least three, amino acids linked together by peptide bonds. Examples of suitably modified PBAEs are described in WO2014/136100 and WO2016/116887.

In some embodiments, the oligopeptide peptide comprises one, two, three, four, five, or more amino acids selected from the group consisting of arginine (R), glutamic acid (E), lysine (K), aspartic acid (D), histidine (H), and cysteine (C). In some embodiments, the oligopeptide contains one, two, three, or more amino acids that are positively charged (such as R, K, or H), or at least partially positively-charged, under physiological conditions. In other embodiments, the oligopeptide contains one or more negatively-charged amino acids (such as D or E), either in the absence of positively-charged amino acids or mixed with positively-charged amino acids. Data shown in the Examples reveal that inclusion of negatively-charged amino acids improves the performance of bald lentiviral vectors, probably by improving endosomal escape. The oligopeptides at each end of the polymer or the polymers comprising the mixture of polymers are preferably identical.

The PBAE nanoparticle ensures that the vector remains intact during transit through the circulation of the patient. The nanoparticle can assist in shielding the viral vector from the host's immune system, for example, ensuring the claimed nanoparticles can maintain high transduction efficiency even in the presence of anti-viral envelope neutralizing antibodies. Furthermore, the particle can ensure that the vector transfects only the desired immune cells: the nanoparticle modifications and/or the presence of targeting moieties can direct the nanoparticle specifically to the tissues of interest.

Preferably, the PBAEs employed in the present technology have a molecular weight from 1,000 to 100,000 g/mol, more preferably from 2,000 to 50,000 g/mol, and even more preferably from 5,000 to 40,000 g/mol.

The polymer layer or shell which coats the viral vector envelope can form a single homogenous polymer layer, or it can form two or more overlapping layers of the same or different polymers, or polymers having different net charge or degree of crosslinking. Other non-PBAE polymers can be intermixed with PBAE or added above or below a PBAE layer to form the polymer shell.

The surface charge (or surface potential or zeta potential) of the nanoparticles is important for tissue targeting and cell transduction and can be finely tuned via polymer modifications. Preferably, the zeta potential of the nanoparticles under physiological conditions of use is in the range from about -30 mV to about +30 mV, or more preferably in the range from about -15 mV to about +15 mV. The polymeric nanoparticle can include one type of polymer or more than one type of polymer. In some embodiments, the polymer coating forms a single layer coating, a dual layer coating, or a multilayer coating. Figure 2 shows an embodiment of the vector nanoparticle that includes first layer 30, second layer 31, and third layer 32 of different polymeric coatings encapsulating the retroviral vector. In some embodiments, the multilayer coating includes an anionic and a cationic polymer. In some embodiments, the cationic polymer includes one or more PBAEs. In some embodiments, the anionic polymer is polyglutamic acid (PGA), chondroitin sulfate and/or hyaluronic acid. In certain embodiments, the viral vector is encapsulated within two or more layers of polymers, such as alternating layers having alternating net electrical charges, i.e., a positively-charged layer is followed by a negatively-charged layer, and then for a positively-charged layer. In some embodiments, the net charge of the nanoparticle is positive at pH 7. In some embodiments, the net charge of the nanoparticle is negative at pH 7. In some embodiments, the net charge of the nanoparticle is tissue-dependent. In some embodiments, the particle is negatively charged, but in the tumor microenvironment it becomes positively charged. In some embodiments, the particle responds to the weakly acidic tumor microenvironment and endo/lysosome through the disintegration or cleavage of certain chemical bonds, by which the PBAE layer underneath is exposed and exerts a "proton-sponge" effect.

As used herein, the term "nanoparticles" refers to a solid particle with a diameter of from about 1 to about 1000 nm. The mean diameter of the nanoparticles of the present technology can be determined by methods known in the art, preferably by dynamic light scattering. In particular, the technology relates to nanoparticles that are solid particles with a diameter of from about 1 to about 1000 nm when analyzed by dynamic light scattering at a scattering angle of 90° and at a temperature of 25°C, using a sample appropriately diluted with filtered PBS and a suitable instrument such as one of the ZETASIZER instruments from Malvern Instruments (UK). Where a particle is said to have a diameter of x nm, there will generally be a distribution of particles about this mean, but at least 50% by number (e.g. >60%, >70%, >80%, >90%, or more) of the particles will have a diameter within the range x ± 20%.

The diameter of the nanoparticle can be from about 5 nm to about 999 nm, and preferably it is from about 50 nm to about 400 nm, more preferably from about 100 nm to about 300 nm. Alternatively, the diameter of the nanoparticle can be from about 10 to about 100 nm. In one embodiment, the nanoparticles exhibit a degree of agglomeration of less than 10%, preferably less than 5 %, and preferably less than 1%. Preferably the nanoparticles are substantially non-agglomerated, as determined by transmission electron microscopy or another method.

### Retroviral vectors

Retroviruses are RNA viruses that possess the ability to integrate into the host genome in a stable fashion. In preferred embodiments, the retroviral vector in the gene delivery vehicle is a lentiviral vector. Lentiviral vectors are derived from lentiviruses, which represent a genus of slow viruses of the Retroviridae family. Lentiviruses include human immunodeficiency viruses (HIV), simian immunodeficiency virus (SIV), equine infectious encephalitis virus (EIAV), caprine arthritis encephalitis virus (CAEV), bovine immunodeficiency virus (BIV) and feline immunodeficiency virus (FIV). Lentiviruses can integrate into the genome of dividing and non-dividing cells. Further, they can persist indefinitely in their hosts and replicate continuously. In some embodiments described herein, the lentiviral vector has a capsid but lacks envelope protein. Preferably, the vector is non-replicative in cells of the intended host or treatment subject. Preferably, the vector does not transduce or alter gene expression of non-targeted cells. Preferably, the vector lacks any genes that cause virulence, particularly virulence manifested as inducing the unintended cell death of non-targeted cells.

Lentiviral vectors exhibit several advantages as tools for immunotherapy. They are less-toxic compared to other vectors and are capable of transducing non-dividing cells, including dendritic cells (He et al. 2007, Expert Rev Vaccines, 6(6):913-24). However, Lentiviruses are more complex than other retroviruses due to the presence of accessory genes (e.g., vif, vpr, vpu, nef, tat, and rev), which are required for replication in vivo.

Bioproduction of integrative but replication-defective lentiviral vectors is based on the separation of cis- and trans-acting sequences of the lentivirus. Transduction in non-dividing cells requires the presence of two cis-acting sequences in the lentiviral genome, the central polypurine tract (cPPT) and the central termination sequence (CTS). This leads to the formation of a triple-stranded DNA "flap", which maximizes the efficiency of gene import into the nuclei of non-dividing cells, including dendritic cells (DCs) (Zennou et al., 2000, Cell, 101(2) 173-85; Arhel et al., 2007, EMBO J, 26(12):3025-25 37). Furthermore, removal of the LTR U3 sequence has resulted in "self-inactivating" vectors which are entirely devoid of viral promoter and enhancer sequences and are safer.

Lentiviral particles, which contain lentiviral vectors, can be produced by transient transfection of HEK 293 cells (with or without the T antigen, adherent or grown in suspension) using a combination of DNA plasmids, for example: (i) a packaging plasmid expressing Gag, Pol, Rev, Tat, and optionally structural proteins or enzymes necessary for packaging of the transfer construct; (ii) a proviral transfer plasmid containing an expression cassette and HIV cis-acting factors necessary for packaging, reverse transcription, and integration; and (iii) a plasmid encoding an envelope protein, such as the glycoprotein of vesicular stomatitis virus (VSV-G), a protein that allows the formation of mixed particles (pseudotypes) that can transduce a wide variety of cells, especially antigen-presenting cells (APCs), including dendritic cells (DCs) and macrophages. In the present technology, the plasmid encoding an envelope protein is omitted completely in certain embodiments, while in other embodiments it is included but its expression is maintained at a low level. Lentiviral particle vectors can also be produced continuously by stably inserting the packaging genes and proviral coding DNA into the cellular genome. A combination of integrated plasmids and transient transfection also can be used. It should be noted, however that, in certain embodiments, lentiviral vectors suitable for use in the gene delivery vehicle described herein are devoid of envelope protein.

Non-integrating lentiviral vectors lacking the envelope protein also can be used in the gene transfer vehicles. Examples of non-integrating lentiviral vectors are found in Coutant et al., PLOS ONE 7(11):e48644 (2102); Karwacz et al., J. Virol. 83(7):3094-3103 (2009); Negri et al., Molecular Therapy 15(9):1716-1723 (2007); and Hu et al., Vaccine 28:6675-6683 (2010).

As the 5'LTR of the proviral sequence is devoid of U3, the expression of the transgene is driven by an internal promoter. Viral promoters, such as the CMV promoter, preferably are not used because of the presence of strong enhancers. Ubiquitous promoters can be used, such as promoters for the human genes encoding ubiquitin, PGK, β-actin, GAPDH, β-kinesin, and the like. Alternatively, promoters active in T-cells and APCs can be used, such as the promoter for human MHC class I genes. In all cases, the promoter preferably does not contain an enhancer. The promoter is preferably selected so as to achieve a therapeutic level of expression of the transgene in the target cells. In some embodiments, the promoter is specific for the target cells, i.e., it enables a therapeutic expression level of the transgene in the target cells, and preferably enables a higher level of transgene expression in target cells than in non-target cells; In certain embodiments, the promoter allows little or no expression of the transgene in non-target cells.

### Polymer Encapsulation of Viral Vectors

Previous technologies using polymer-encapsulated vehicles for transfecting cells have involved plasmids or other nucleic acid molecules that are directly coated with polymers such as PBAE. Some such technologies have achieved transfection using nucleic acid molecules (US2016/0145348A1, Mangraviti et al. 2015, Anderson et al. 2004, WO2016/116887). Poor stability of PBAE polymers at pH 7 has been reported (Lynn et al. 2000). Such preparations may cause cell toxicity, resulting in cell death upon exposure to polymer-coated plasmids or other nucleic acid molecules. Therefore, the dose of PBAE-encapsulated vector administered to cells in vivo or in vitro should be kept to the minimum needed for useful transduction, so as to avoid toxic effects. However, PBAE-encapsulated viral vectors advantageously resist degradation at low pH.

To the knowledge of the present inventors, polymer encapsulation of viral vectors such as LV has not been previously accomplished. The inventors have developed methods for polymer encapsulation of LV and other viral vectors that result in stable polymer-encapsulated LV particles with low toxicity and high effectiveness at transducing cells. Important factors in producing viable encapsulated vectors include the type of polymer used (e.g., its charge and charge distribution), the ratio of polymer molecules to vector particle, and the pH used for encapsulation. The effect of these parameters was investigated and is described in Examples 1 and 2 below.

Using PBAE polymers and LV, successful encapsulation, resulting in particles capable of efficient transduction of target cells, requires about 10⁸ to 10¹¹ polymer molecules per vector particle; higher ratios can produce toxicity. Further, the optimum pH for successful encapsulation differs depending on whether the LV contains VSV-G⁺ or not (VSV-G⁻ or "bald" LV). For VSV-G⁺ LV, optimum encapsulation with PBAE occurs at about pH 5, as indicated by the high level of VSV-G antibody-resistant transduction at pH 5, while little or no encapsulation occurred at higher pH values. In contrast, for VSV-G⁻ LV, optimum PBAE encapsulation efficiency occurs over a broader and lower pH range, such as about 5.0, 5.5, 6.0, or 6.5, or 5.0-6.5, or 5.0-6.0, or 5.5-6.0, or 5.5-6.5. whereas for VSV-G- LV, optimum PBAE encapsulation efficiency occurs at a lower pH range, such as about 5.0, 5.5, 6.0, or 6.5, or 5.0-6.5, or 5.0-6.0, or 5.5-6.0, or 5.5-6.5. See Example 2. Finally, the optimum pH for successful encapsulation that does not impact viability of transduced cells is the same whether the LV contains VSV-G⁺ or not, such as 5.0 or 5.5.

The nanoparticles can be formed by mixing a solution comprising the required materials and then incubating the solution. For example, nanoparticles can be formed by mixing a solution comprising PBAE with a solution comprising the viral vector, then incubating the solution. In some embodiments, the solution contains a sugar, such as glucose. In some embodiments the solution contains 5% glucose. In some embodiments, the solution contains a buffer. In some embodiments, the buffer is sodium acetate, and the final concentration of sodium acetate buffer is from 2 to 50 mM, from 10 to 30 mM, or about 25 mM. In some embodiments, the buffer is Tris-HCl buffer, and the final concentration of Tris-HCl buffer is from 2 to 40 mM, from 5 to 30 mM, or from 15 to 25 mM. In some embodiments, the solution is incubated for at least 5 minutes. In some embodiments, the solution is incubated for at least 30 minutes. In some embodiments, the solution is incubated overnight. The solution can be incubated at 4 to 40°C, or at 17 to 25°C; preferably, the solution is incubated at 4°C.

In some embodiments, the nanoparticles are formed using layer-by-layer deposition of ionic polymers onto retrovirus particles, thus forming multilayer-coated viral vectors. In some embodiments, a solution containing ionic polymers is contacted with a solution containing viral vector particles. The ratio of polymer to viral vector particles can be in the range from about 1:100 to about 5:1 by weight. The ratio can be varied so as to ensure that the nanoparticles contain on average a single viral vector particle per polymer-coated nanoparticle. For example, the weight ratio of all polymers to viral vector can be from about 1:100 to about 1:1, or from about 1:50 to about 1:1, or from about 1:20 to about 1:1, or from about 1:10 to about 2:1, or from about 1:1 to about 5:1. In some embodiments, one or more polymers are added to a suspension of retrovirus particles in glucose or another sugar compatible with lyophilization and direct injection after reconstitution into a human or other subject. In some embodiments, negatively-charged and positively-charged polymer are added sequentially to form polymer layers having alternating charges. In some embodiments, the polymers are added at 30 min intervals. In some embodiments, there are five layers of polymers. In some embodiments, the multilayer-coated viral vectors have a net negative charge at pH 7. In some embodiments, the multilayer-coated viral vectors have a net positive charge at pH 7. The number and polarity of layers can be chosen so as to optimize transduction efficiency of particular cell types of interest.

Preferably, nanoparticles are formed at a pH at which the viral vector particle surface charge, surface potential, or zeta potential is opposite to that of the one or more polymers that form the polymer shell around the viral vector particle. In some embodiments, the pH is above that of the isoelectric point of one or more retroviral envelope proteins, or the envelope as a whole. For example, the pH can be at or above about 4.0, 4.5, 5.0, 5.4, 5.4, 5.5, 5.6, 5.8, 6.0, 6.2, 6.4, 6.5, 6.8, or 7.0. In some embodiments, the nanoparticles are formed in a solution having a pH of about 5.

### Targeting Moieties

The nanoparticle vector delivery vehicle can be targeted to specific target cells or tissues requiring therapy, or in which protein expression by the vector is desired. Another embodiment of targeting moiety is shown in Fig. 3, in which the delivery vehicle contains targeting moieties covalently or non-covalently attached to the polymer surface. Targeting moieties can target specific cells or classes of cells either *in vivo* or *in vitro.* Examples of targeting moieties include proteins (e.g., antibodies, including human, mouse, camelid and shark antibodies, single chain antibodies, nanobodies, diabodies, and antigen-binding fragments of antibodies), peptides, nucleic acids such as aptamers or oligonucleotides, or ligands that bind to cell-surface receptors or components of extracellular matrix.

In one embodiment, the delivery vehicle is targeted in particular to T-cells for expression of one or more therapeutic proteins, such as chimeric antigen receptor (CAR) proteins, so as to enhance the killing of cells (e.g., tumor cells or pathogen cells) to which the chimeric receptor binds. In a preferred embodiment, the target cells for the vector are T-cells (as CD3+ T cells) or NK cells. Targeting can be accomplished using one or more DNA aptamers or antibodies having specificity for a protein on the surface of the target cells. The aptamer or antibody serves as a targeting moiety when integrated into the nanoparticle and exposed at the surface of the nanoparticle. For example, the targeting moiety can specifically bind CD3 or CD8, or a combination of CD3 and CD8. Alternatively, or in addition thereto, the targeting moiety can specifically bind to a surface protein found only on naive T-cells, such as CD45RA, CCR7, CD62L, CD27, CD28, IL7-Ra, or DC-SIGN. Combinations of these targeting moieties also can be used.

Targeting is not limited to CAR technology or hematologic cells, however. Any cell type can be targeted. For example, in certain gene therapy applications, a specific tissue or cell type (e.g., hematopoietic cells, myocytes, islet cells, etc..) may be targeted according to the need to repair or replace a defective function. In other applications, quiescent cells may be targeted over dividing cells so as to reduce risk of recombination events). In still other applications, dividing cells such as cancer cells may be targeted, for example in a cancer vaccine.

### Transgenes and Their Expression Products

The viral vector in the gene delivery vehicle described herein contains one or more transgenes which, after entering a cell, become expressed in the cell and produce expression products. The viral vector can contain one, two, three, or more transgenes. The transgenes are nucleic acid molecules that encode any naturally occurring or artificial (i.e., recombinant) protein, polypeptide, or peptide, including a therapeutic protein or a marker protein. Such vector-expressed proteins preferably have a therapeutic value in the subject in which they are expressed, and are thus therapeutic proteins. One type of therapeutic protein is a chimeric antigen receptor (CAR). CARs for use in the technology described herein are recombinant antigen receptors, such as T-cell receptors, with binding specificity for cell surface antigens that do not require peptide processing or HLA expression in order to bind. The antigen-binding portion can be an scFv derived from an antibody, from an Fab selected from a library, or can be a naturally occurring ligand for a cell surface receptor. It also can be a nanobody (VHH). The remainder of the CAR molecule couples the antigen binding domain to intracellular signal transduction domains. Specifically, a CAR of the technology includes one or more of each of the following domains: an extracellular antigen-binding domain, a hinge and transmembrane domain, and intracellular signaling domains (CD3zeta, and/or CD28 or 41-BB/OX40). Further details of CARs suitable for use in the gene delivery vehicle and sequences to encode them in a lentiviral vector are found in WO2016/012623A1.

Other genes that can be delivered using the gene delivery vehicles of the present technology include interleukins and cytokines, such as interleukin 1 (IL-1), IL-2, IL-3, IL-4, IL-5, IL-6, IL-7, IL-8, IL-9, IL-10, IL-11 IL-12, GM-CSF, and G-CSF. Genes encoding antigens such as viral antigens, bacterial antigens, fungal antigens or parasitic antigens also can be delivered. Viruses whose antigens can be expressed include picornavirus, coronavirus, togavirus, flavirvirus, rhabdovirus, paramyxovirus, orthomyxovirus, bunyavirus, arenvirus, reovirus, retrovirus, papovavirus, parvovirus, herpesvirus, poxvirus, hepadnavirus, and spongiform virus. Preferred viral targets include influenza, herpes simplex virus 1 and 2, measles, small pox, polio or HIV. Pathogens include trypanosomes, tapeworms, roundworms, helminths. Tumor antigens such as fetal antigen or prostate specific antigen also can be targeted in this manner. Administration of a vector according to the present technology for vaccination purposes can be performed in accordance with any known vaccination strategy. Vaccination of an individual can be carried out according to any desired schedule; preferably vaccination is only required infrequently, such as yearly or biennially, and provides long term immunologic protection against the infectious agent.

A viral vector transgene also can encode an anti-checkpoint protein. Examples of anti-checkpoint proteins that can be encoded by the transgene are inhibitors of CTLA-4, PD1, PDL1, LAG-3, TIM 3, B7-H3, ICOS, IDO, 4-1BB, CD47, and combinations thereof.

### Uses of the Gene Delivery Vehicles

The gene delivery vehicles of the present technology can be used in any application that requires effective transduction of genetic material in cells of interest, such as mammalian cells or human cells by means of a retroviral vector, such as a lentiviral vector. The delivery vehicles transduce cells with high efficiency and exhibit low toxicity and low immunogenicity. They can permanently integrate genetic material into the host cell genome, making the system particularly suitable for permanent labeling of cells and their progeny. Since the vehicles present high specificity, low toxicity, and high transduction capability even *in vivo,* they are particularly suitable for gene transfer and gene therapy applications both for therapy in humans and animals, as well as in animal models, thus allowing investigation of the molecular mechanisms underlying different biological functions and their translation into therapy. However, some preparations of polymer-encapsulated viral vector nanoparticles may be limited to transducing certain cell types, such as preferentially transducing dividing cells and not transducing non-dividing cells.

The delivery vehicles of the present technology can be used to genetically engineer cells for biotechnology applications, such as biologics manufacturing and livestock and plant improvements. Precise and stable insertion of the gene or genes of interest is essential for these activities, and can be consistently achieved with the vehicle of the technology. The vehicles can be used to insert genes not originally present in cells used for manufacturing purposes, such as transformed cells (Chinese hamster ovary (CHO) cells, HEK293 cells) or primary cells. Alternatively, the vehicles can be used to "knock out" certain genes present in those cells, such as genes involved in protein glycosylation, a common cause of immunogenicity. The vehicles may, for example, be used to prevent, treat or cure diseases that affect livestock, or to improve their economic value, by accelerating growth and maturation.

The gene delivery vehicle can be used to deliver genetic material as part of *in vitro* or *in vivo* gene therapy. The gene delivery vehicle can be employed in gene therapy to replace a disease-causing mutated version of a gene with a healthy copy of the gene; inactivate or "knock out" a gene that is not functioning properly, and/or to introduce a new gene in a cell, such as to fight a disease. The use of retroviral vectors, in general, allows for permanent insertion of genetic material into a cell of interest. The use of lentiviral vectors, in particular, allows for permanent insertion of genetic material even into non-dividing cells.

The gene delivery vehicles are capable of transducing cells *in vivo,* leading to functional expression of the transgene and, therefore, can be used to conduct gene therapy. The vector delivery vehicle can be administered parenterally, such as intravenously, intramuscularly or intra-tumor. Alternatively, the vector delivery vehicle can be administered externally, such as orally or intra nasally. The gene delivery vehicle can be administered by any other suitable route, including intracerebral and pulmonary.

The gene delivery vehicles of the present technology are also capable of transducing cells *in vitro,* leading to functional expression of the transgene and, therefore, can be used to conduct gene therapy *in vitro* and *ex vivo.* Patient's cells can be obtained (e.g., via blood or bone marrow sample, or biopsy) and contacted with one of the described vector delivery vehicles in a laboratory setting. The transduced cells can then be returned to the patient, and help treat a disease.

Diseases that can be treated with gene therapy using gene delivery vehicles of the technology include inherited disorders, such as severe combined immunodeficiency (SCID), chronic granulomatous disease (CGD), Wiskott-Aldrich Syndrome (WAS), relapsed acute 30 lymphoblastic leukemia (ALL), adrenoleukodystrophy (ALD), hemophilia B, adenosine deaminase (ADA) deficiency, cystic fibrosis (CF), beta-thalassemia, sickle cell disease, Duchenne muscular dystrophy, familial hypercholesterolemia, and hereditary blindness. Treatable diseases also include neurodegenerative disorders, such as Parkinson's Disease, Alzheimer's Disease and Huntington's Disease. They further include acquired diseases such as heart disease, diabetes, osteoarticular disorders, and infectious diseases (e.g., influenza, HIV, hepatitis, among others). Further treatable diseases include cancer, such as cancers of the prostate, pancreas, brain, skin, liver, colon, breast, and kidney.

### Example 1. Production of Polymer-Coated Lentiviral Vectors.

Polymer-coated lentiviral vectors for use in cell transduction studies were made using the following materials and methods.

### Materials

The transfer vector plasmid was pARA-CMV-GFP or pARA-CMV-mCherry or pARA or pAra-hUBC-CD19.

A kanamycin-resistant plasmid encoded for the provirus (a non-pathogenic and non-replicative recombinant proviral DNA derived from HIV-1, strain NL4-3), in which an expression cassette was cloned. The insert contained the transgene, the promoter for transgene expression and sequences added to increase the transgene expression and to allow the lentiviral vector to transduce all cell types including non-mitotic ones. The coding sequences corresponded to the gene encoding Green Fluorescent Protein (GFP) or mCherry (red fluorescent protein) or CD19. The promoter was the human ubiquitin promoter or the CMV promoter. It was devoid of any enhancer sequence and it promoted gene expression at a high level in a ubiquitous manner. The non-coding sequences and expression signals corresponded to Long Terminal Repeat sequences (LTR) with the whole cis-active elements for the 5'LTR (U3-R-U5) and the deleted one for the 3'LTR, hence lacking the promoter region (ΔU3-R-U5). For the transcription and integration experiments, encapsidation sequences (SD and 5'Gag), the central PolyPurine Tract/Central Termination Site for the nuclear translocation of the vectors, and the BGH polyadenylation site were added.

The packaging plasmid was pARA-Pack. The kanamycin resistant plasmid encoded for the structural lentiviral proteins (GAG, POL, TAT and REV) used in trans for the encapsidation of the lentiviral provirus. The coding sequences corresponded to a polycistronic gene gag-pol-tat-rev, coding for the structural (Matrix MA, Capsid CA and Nucleocapside NC), enzymatic (Protease PR, Integrase IN and Reverse Transcriptase RT) and regulatory (TAT and REV) proteins. The non-coding sequences and expression signals corresponded to a minimal promoter from CMV for transcription initiation, a polyadenylation signal from the insulin gene for transcription termination, and an HIV-1 Rev Responsive Element (RRE) participating for the nuclear export of the packaging RNA.

The envelope plasmid, when used, was pENV1. This kanamycin-resistant plasmid encoded glycoprotein G from the Vesicular Stomatitis Virus (VSV-G) Indiana strain, used for the pseudotyping of some of the lentiviral vectors. The VSV-G genes were codon optimized for expression in human cells, and the gene was cloned into pVAX1 plasmid (Invitrogen). The coding sequences corresponded to codon-optimized VSV-G gene, and the noncoding sequences and expression signals corresponded to a minimal promoter from CMV for transcription initiation, and the BGH polyadenylation site to stabilize the RNA.

### Production of VSV-G⁻ ("Bald") Lentiviral Vector Particles

LV293 cells were seeded at 5 ×10⁵ cells/mL in 2 × 3000 mL Erlenmeyer flasks (Corning, 431252) in 1000 mL of LVmax Production Medium (Gibco, A3583401). The two Erlenmeyers were incubated at 37°C, 65 rpm under humidified 8% CO₂. The day after seeding, the transient transfection was performed. PEIPro transfectant reagent (PolyPlus, 115-010) were mixed with transfer vector plasmid (pARA-CMV-GFP or pARA-CMV-mCherry or pARA or pAra-hUBC-CD19) and packaging plasmid (pARA-Pack). After incubation at room temperature, the mix PEIPro/Plasmid was added drop by drop to the cell line and incubated at 37°C, 65 rpm under humidified 8% CO₂. At day 3, the lentivector production was stimulated by sodium butyrate at 5 mM final concentration. The bulk mixture was incubated at 37°C, 65 rpm under humidified 8% CO₂ for 24 hours. After clarification by deep filtration at 5 and 0,5 µm (Pall, 609-122U), the clarified bulk mixture was incubated 1 hour at room temperature for DNase treatment.

Lentivector purification was performed by chromatography (Q mustang membrane) and eluted by NaCl gradient. Tangential flow filtration was performed on a 100 kDa HYDROSORT membrane (Sartorius, 3M81446802E-SW), which allowed to reduce the volume and to formulate in specific buffer at pH 7, ensuring at least 2 years of stability. After sterile filtration at 0.22 µm (Millipore, SLGVM33RS), the bulk drug product was filled in 2 mL glass vials with aliquots less than 1 ml, then labelled, frozen and stored at < -70°C.

The bald LV number was evaluated by physical titer quantification. The assay was performed by detection and quantitation of the lentivirus associated HIV-1 p24 core protein only (P24 ELISA Cell Biolabs QUICK TITER Lentivirus Titer Kit).

### Production, purification and quantification of 5 PBAE Polymer

Poly(β-amino ester) polymers were synthesized following a two-step procedure similar to that described by Dosta et al. 5-amino-1-pentanol (Sigma-Aldrich 3.9 g, 38 mmol) and 1-Hexylamine (Sigma-Aldrich 3.9 g, 38 mmol) were mixed in a round bottom flask and combined with 1,4-butanediol diacrylate (Aldrich 18 g, 82 mmol) in a 2.1:1 molar ratio of diacrylate to amine monomers. The mixture was heated to 90 °C with stirring for 20 h. Then it was cooled to room temperature to form a light yellow viscous solid oil and stored at -20 °C until further use.

Synthesized structures were confirmed by 1H-NMR spectroscopy. NMR spectra were recorded in a 400 MHz Varian (Varian NMR Instruments, Claredon Hills, IL) and methanol-d4 was used as solvent. Molecular weight determination was conducted on an HPLC Elite LaChrom system (VWR-Hitachi) equipped with a GPC SHODEX KF-603 column (6.0 × about 150 mm), and THF as mobile phase. The molecular weight was calculated by comparison with the retention times of polystyrene standards. Weight-average molecular weight (Mw) was 4400, and number-average molecular weight (Mn) was 2900.

Oligopeptide-modified PBAEs were obtained by end-modification of acrylate-terminated polymer C6 with thiol-terminated oligopeptide at 1:2.5 molar ratio in dimethyl sulfoxide (DMSO). The following synthetic procedure to obtain tri-arginine end-modified PBAE polymer, C6-CR3, is shown as an example: a solution of C6 (113 mg, 0.054 mmol) dissolved in DMSO (1.1 mL) and a solution of hydrochloride H-Cys-Arg-Arg-Arg-NH₂ (CR3 - 95 % purity - purchased from Ontores) (99 mg, 0.13 mmol) in DMSO (1 mL) were mixed in a teflon-lined screw cap vial and stirred in a water bath with a controlled temperature of 20°C for 20 h. The capped polymer was precipitated with 5 volumes of diethyl ether. After centrifugation (10 min at 3000 g), the polymer was washed with 2 volumes of fresh mixture of diethyl ether and acetone (7:3 v/v), then the residue was dried under vacuum before it was formed into a stock of 100 mg/mL in DMSO, which was stored at -20 °C.

In a further example, tri-lysine end-modified PBAE polymer, C6-CK3 was obtained by mixing a solution of C6 (100 mg, 0.048 mmol) dissolved in DMSO (1.1 mL) and a solution of hydrochloride H-Cys-Lys-Lys-Lys-NH₂ (CK3) (78 mg, 0.12 mmol) in DMSO (1.8 mL) and processing the coupling reaction as previously described for C6-CR3. For the tri-histidine end-modified PBAE polymer, C6-CH3 the solution of C6 (65 mg, 0.031 mmol) dissolved in DMSO (1.1 mL) that was mixed with a solution of hydrochloride H-Cys-His-His-His-NH2 (CH3) (53 mg, 0.078 mmol) in DMSO (1.2 mL). The tri-aspartate end-modified PBAE polymer, C6-CD3 the solution of C6 (96 mg, 0.046 mmol) dissolved in DMSO (1.1 mL) that was mixed with a solution of hydrochloride H-Cys-Asp-Asp-Asp-NH2 (CD3) (96 mg, 0.046 mmol) in DMSO (1.7 mL). Finally, the tri-glutamate end-modified PBAE polymer, C6-CE3 the solution of C6 (280 mg, 0.13 mmol) dissolved in DMSO (1.1 mL) that was mixed with a solution of hydrochloride H-Cys-Glu-Glu-Glu-NH2 (CE3) (184 mg, 0.34 mmol) in DMSO (4.6 mL).

The chemical structure of the oligopeptide-modified PBAEs was confirmed by 1H-NMR spectroscopy by the disappearance of acrylate signals and the presence of signals typically associated with amino acid moieties. Residual free peptide content was quantified by UV detection (wavelength 220 nm) after separation by UPLC ACQUITY system (Waters) equipped with a BEH C18 column (130 A, 1.7 µm, 2.1x50 mm, temperature 35 °C) using an acetonitrile gradient.

### Production of Fab-PGA

15 kDa polyglutamic acid (PGA) (Alamanda Polymers) was dissolved in MES buffer pH 6.0 to 20 mg.mL⁻¹ then sonicated for 10 min in a bath sonicator. 1.8 mL of PGA solution was added to 0.9 mL of ethyl-N'-(3-dimethylaminopropyl)carbodiimide-HCl (4 mg.mL⁻¹, 16 equiv.) and 0.9 ml of sulfo-NHS (2,75 w/w EDC) both in MES buffer pH 6.0 and the solution was mixed at room temperature for 15 min. The resulting activated PGA was added to a solution of antimouse CD3ε F(ab')2 fragment (InVivoMAb) in phosphate-buffered saline (PBS) at a 4:1 molar ratio and mixed at room temperature for 2 h. Excess reagents and unlinked PGA were removed by filtration (30,000 MWCO Vivaspin column) and the buffer was exchanged 7 times against PBS. Antibody concentration was determined using a NanoDrop 2000 Spectrophotometer (Thermo Scientific).

### Stability Kinetics of VSV-G⁺ LV as a Function of pH

10 µL of Drug Product pseudotyping Lentivectors or bald lentivectors were diluted in 10 µL of different buffers (pH 5 to 7) during different times (0, 15, 30, 60, 90 and 120 minutes) at +4°C.

Titration for the pseudotyping Lentivectors was performed by flow cytometry as follows. HEK293T cells (8×10⁵ cells/well) were seeded in 24-well plates and incubated at 37°C in a 5% CO₂ humidified atmosphere. After 4 h, the culture medium was removed and 300 µL of fresh DMEM containing 10% FBS, 1% PS and 10 µL ow each condition were added to each well. After 2 h, each well was supplemented with 0.5 mL fresh culture medium. Three days after infection, the HEK293T cells in each well were trypsinized, fixed (BD Cell FIX solution #340181) and the number of fluorescence-positive cells was determined using flow cytometry (AttuneNXT; Invitrogen, Inc.).

The following formula was used to convert the percentage of GFP-expressing cells for a specific dilution into TU: TU/ml = (% of cells expressing eGFP/100) × total number of HEK293T cells at time of infection/volume of virus stock added (mL).

The titration for the bald lentivectors was performed by physical titer quantification. The assay was performed by detection and quantitation of the lentivirus associated HIV-1 p24 core protein only (P24 ELISA Cell Biolabs' QUICK TITER Lentivirus Titer Kit). A pre-treatment samples allows to distinguish the free p24 from destroyed Lentivectors.

### Encapsulation of VSV-G⁺ LV and Bald LV with Oligopeptide-Modified PBAE

Encapsulation of lentiviral vectors was performed as follows. Lentiviral vectors were diluted in 25 mM citrate buffer pH 5.0 (or in an appropriate buffer system at a different defined pH) to prepare a final volume of 75 µL per replicate. PBAE polymers were diluted in the same buffer as for lentiviral vectors (75 µL per replicate) and vortexed 2 s for homogenization. The diluted polymers were added to the diluted vectors in a 1:1 ratio (v/v), the mixes were gently vortexed for 10 s and incubated 30 minutes at +4 °C. Finally, an equal volume of culture medium (150 µL) was added to the encapsulation reactions before transfer to cells.

### Coating of PBAE-Encapsulated VSV-G⁺ LV and Bald LV with PGA-Fab

The previously described encapsulation method was modified as follows. Lentiviral vectors were diluted in 25 mM citrate buffer pH 5.0 (or in appropriate buffer system at a defined pH) to prepare a final volume of 75 µL per replicate. PBAEs were diluted in the same buffer as for lentiviral vectors (75 µL per replicate) and vortexed 2 s for homogenization. The diluted polymers were added to the diluted vectors in a 1:1 ratio (v/v). The mixes were gently vortexed for 10 s and incubated 30 minutes at +4 °C. PGA-Fab polymer diluted in 25 mM citrate buffer pH 5.0 (or in appropriate buffer system at a defined pH) was added to the encapsulation mix and gently vortexed 10 s for homogenization. The coating reaction mix was incubated 30 minutes at +4 °C and the volume was completed up to 300 µL with culture medium before being transferred to cells.

### Example 2. Zeta Potential Measurements of PBAE-Encapsulated Lentiviral Vectors.

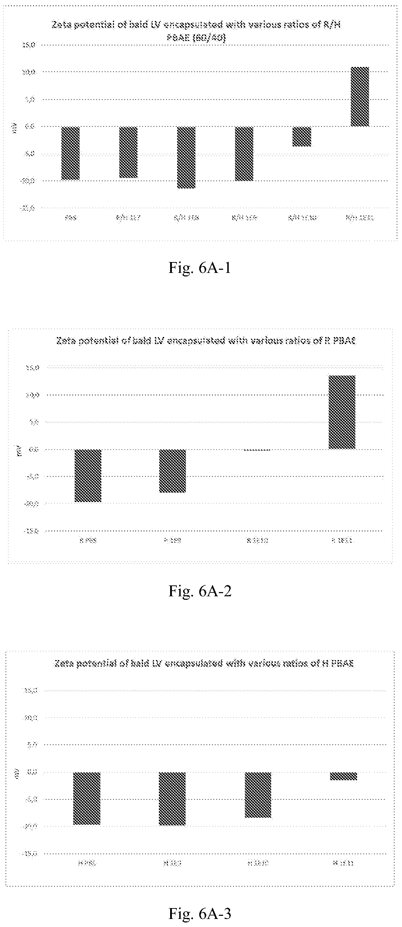

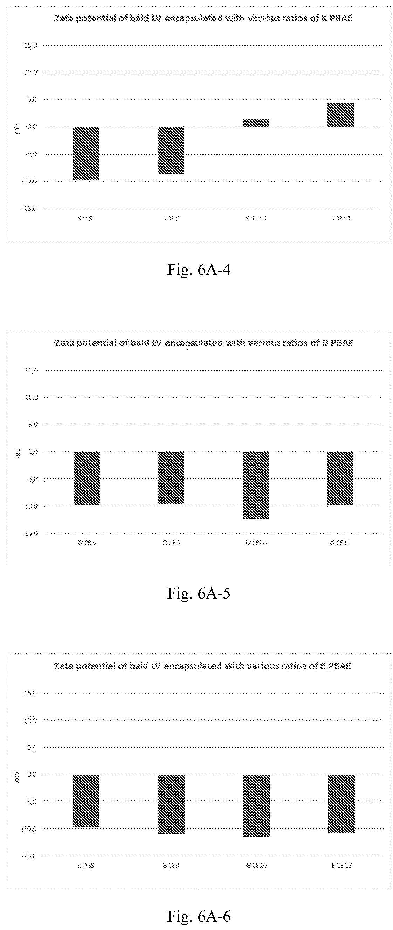

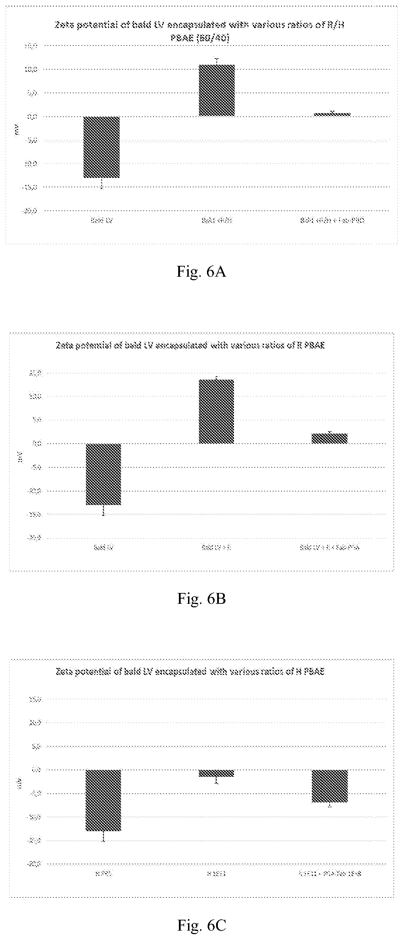

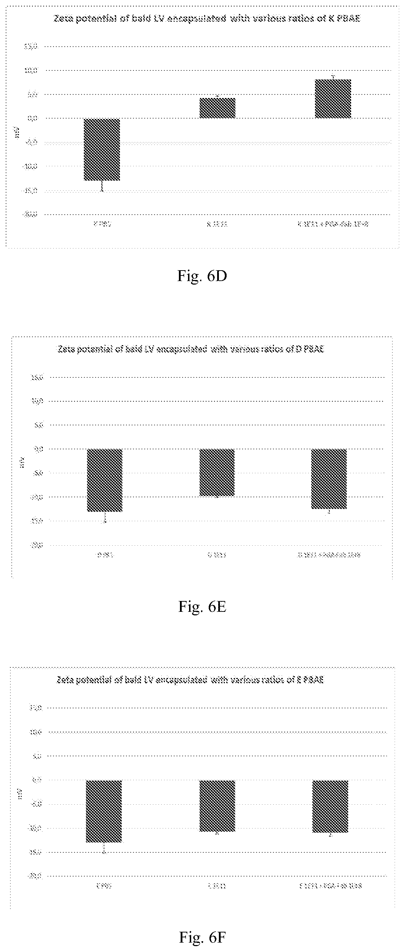

Particle surface charge measurements of the LV/PBAE/PGA-Fab complexes were determined by dynamic light scattering (DLS) at room temperature with a Zetasizer Nano ZS (Malvern Instruments Ltd, United Kingdom, 4-mW laser) using a wavelength of 633 nm). Measurements were carried out by mixing 200 µL of complexes with 800 µL of D-PBS 1x buffer solution (pH 7.1). The results were plotted as mean and standard deviation of 3 measurements analyzed by intensity, and are shown in Figs. 6A-6F. The results showed surface potential modification by the encapsulation of the vector particles with polymer in accordance with the charge of the polymer, indicating successful encapsulation with polymer.

### Example 3. Transduction of HEK293T Cells by Polymer-Coated Lentiviral Vectors.

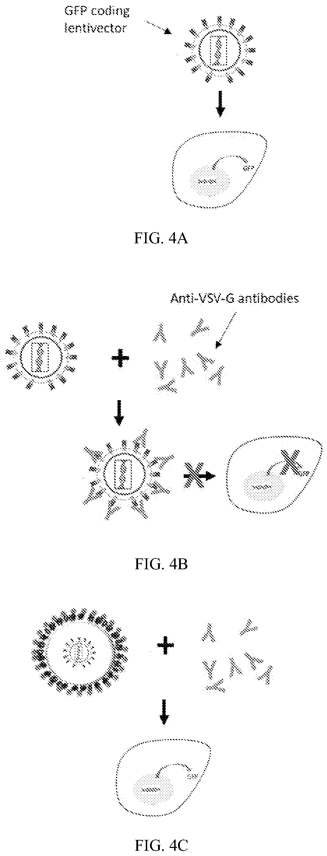

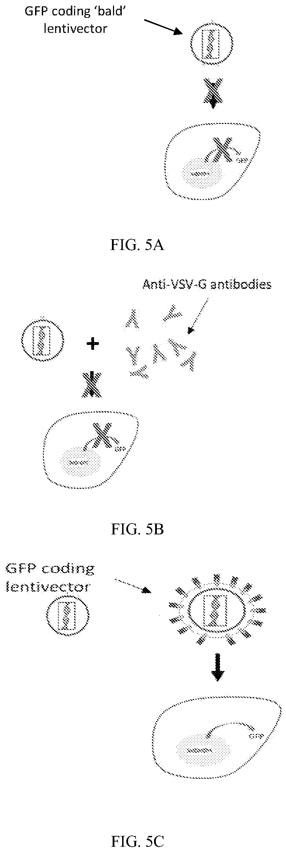

In order to test the effect of polymer coating on the transduction capability of lentiviral vectors, peptide-conjugated poly(beta-amino ester) polymers were prepared and used to coat lentiviral vectors and test transduction of HEK293T cells. The lentiviral vectors carried a green fluorescent protein (GFP) transgene, and transduction was quantified by GFP expression in the transduced host cells (see FIG. 4A). Peptides having different groups of charged amino acids were conjugated to the polymer backbone to test the effect of charge. Vector particles with and without VSV-G protein in their envelopes were polymer coated. The effectiveness of polymer encapsulation was evaluated by determining the transduction ability of the vectors in the presence of antibody to VSV-G, which has the capability to block transduction that is dependent on exposed VSV-G proteins (see FIG. 4B), but will not block transduction by vector that is effectively polymer encapsulated and does not rely on exposed VSV-G for transduction (see FIG. 4C). Similar experiments were performed with LV lacking VSV-G; this strategy is depicted in FIGS. 5A-5C. These "bald LV" were not capable of performing transduction due to the lack of VSV-G protein, but became capable of transduction after their encapsulation by PBAE polymers. Polymer enabled transfection was not expected to be blocked by anti VSV-G Ab.

Encapsulated vectors were prepared as follows. Vector dilutions were prepared in a first set of 1.5mL microtube in a final volume of 150µL per replicate, and the polymer dilutions were prepared in a second set of 1.5mL microtubes with the same final volumes. The diluted polymer was then added to the diluted vector and the mix was homogenized by gently pipetting to avoid any bubbles or foam. Encapsulation mixes were incubated 30 minutes at +4°C.

For the quantification of transduction, cells were seeded in 24-well plates at a density of 8 × 10⁴ cells per well in complete medium containing 10% FBS and incubated for 4 h to adhere. To test neutralization of transduction by anti-VSV-G antibody, 300µL of encapsulated vector was mixed either with 300µL of complete cell culture medium or with 300µL of polyclonal anti-VSVg rabbit serum diluted 100-fold in complete cell culture medium, and incubated 30 minutes at 37°C, 5%CO₂. Cells were then transduced by replacing the medium with 300 µL of vector in antibody neutralization medium or culture medium lacking antibody, followed by incubation at 37°C, 5%CO₂ for 2h. After adsorption, 1mL of complete medium was added to each well. At 72h post transduction, the cells were trypsinized and resuspended in 200µL of Cellfix 1X, and the percentage of cells expressing GFP was determined with an Attune NxT flow cytometer using the BL1 channel.

The polymers used in the encapsulation experiments were poly(beta-amino ester) polymers (PBAE) as shown in the formula below, conjugated to charged peptides (Pep) and optionally cholesterol (R). Polymer R refers to PBAE conjugated to the peptide CRRR (same peptide at both ends). H polymer refers to PBAE conjugated to the peptide CHHH. E polymer refers to PBAE conjugated to the peptide CEEE. D polymer refers to PBAE conjugated to the peptide CDDD. Mixtures of the peptides were tested at different ratios indicated in the tables below. R/H refers to a 60/40 mixture of polymers R and H. Polymers indicated with "chol" were also conjugated to cholesterol as the R group indicated in the structure below. The value of "m" was 3 and the value of "o" was 1.

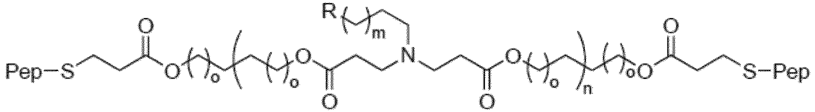

The following tables show the polymer mixtures and ratio of polymer molecules per LV particle used for encapsulation and tested for transduction capability.

**Table 1. Polymer Coatings Used To Test Effect Of Charge.**

| **Influence of the charge on the LV coating (HEK293T)** | |
|---|---|
| ***1^{st} series: Test of negatively charged polymers*** (With pilot LV-GFP batch) | |
| R-polymer mixed with E-polymer: | 100/0 - 66/33 - 33/66 - 0/100 |
| R-chol polymer mixed with E-chol polymer: | 100/0 - 66/33 - 33/66 - 0/100 |
| R-polymer mixed with E-chol polymer: | 100/0 - 66/33 - 33/66 - 0/100 |
| R-chol polymer mixed with E polymer: | 100/0 - 66/33 - 33/66 - 0/100 |
| Test on 3 polymer/LV ratios: 10⁹, 10¹⁰ and 10¹¹, +/- antibody | |

**Table 2. Further Polymer Coatings Used To Test Effect Of Charge.**

| **Improvement of the LV coating with D and K polymers (HEK293T)** | |
|---|---|
| ***2^{nd} series: if negatively charged polymers improve the encapsulation** (*With pilot LV-GFP batch) | |
| R-polymer mixed with E-polymer: | 100/0 - 66/33 - 33/66 - 0/100 |
| R-polymer mixed with D-polymer: | 100/0 - 66/33 - 33/66 - 0/100 |
| R-chol polymer mixed with E-chol polymer: | 100/0 - 66/33 - 33/66 - 0/100 |
| R-chol polymer mixed with D-chol polymer: | 100/0- 66/33 - 33/66 - 0/100 |
| K-polymer mixed with E-polymer: | 100/0 - 66/33 - 33/66 - 0/100 |
| K-polymer mixed with D-polymer: | 100/0 - 66/33 - 33/66 - 0/100 |
| K-chol polymer mixed with E-chol polymer: | 100/0 - 66/33 - 33/66 - 0/100 |
| K-chol polymer mixed with D-chol polymer: | 100/0 - 66/33 - 33/66 - 0/100 |
| Test on the best polymer/LV ratio, +/- antibody | |

**Table 3. Polymer Coatings Used With LV Lacking VSV-G.**

| **Evaluation of the coating of LV without VSV-G (HEK293T)** | |
|---|---|
| ***2^{nd} series: if negatively charged polymers do not improve the encapsulation*** - ***test of LVs without VSV-G*** (With pilot LV-GFP batch and UC batches of LV-GFP +/- VSV-G) | |
| R/H-polymer mixed with E-polymer: | 100/0 - 66/33 - 33/66 - 0/100 |
| R/ H-polymer mixed with D-polymer: | 100/0 - 66/33 - 33/66 - 0/100 |
| R/H-chol polymer mixed with E-chol polymer: | 100/0 - 66/33 - 33/66 - 0/100 |
| R/H-chol polymer mixed with D-chol polymer: | 100/0 - 66/33 - 33/66 - 0/100 |
| Test on 3 polymer/LV ratios: 10⁹, 10¹⁰ and 10¹¹, +/- antibody | |

In order to test the effect of polymer coating on the transduction capability of polymer-coated lentiviral vectors lacking viral envelope protein, peptides having different charged amino acids were conjugated to the polymer backbone, and the resulting polymers were used to coat lentiviral vectors and test transduction of HEK293T cells.

GFP expression data were obtained as flow cytometer results after transduction with vectors having the indicated ratios of polymer molecules per lentiviral vector particle (LV). Under each condition, the addition of polyclonal anti-VSV-G rabbit serum reduced the rate of transduction with a ratio of 10⁹, but Ab failed to reduce transduction at ratios of 10¹⁰ and 10¹¹, indicating that at least about 10¹⁰ polymers per LV particle were needed for effective encapsulation. The data also confirmed that polymer-encapsulated LV were able to effectively carry out transduction, and that positively charged polymer is most effective, with transduction efficacy decreasing with increasing introduction of negative charge in the polymer.

A similar experiment was carried out using cholesterol-conjugated PBAE polymer. The results were similar to polymer that was not conjugated to cholesterol for both R and E polymers.

A similar experiment was carried out using LV that lack the VSV-G envelope protein ("bald LV"). The results indicated that the uncoated bald LV were not able to induce transduction. However, polymer encapsulated bald LV were able to effectively transduce the cells at polymer molecule/LV ratios of 10⁹,10¹⁰, or 10¹¹. As expected, the addition of anti-VSV-G Ab had no effect. Positively charged polymers were much more effective than neutral or negatively charged polymers. Similar results were obtained using cholesterol conjugated polymer, and also for polymer mixtures containing peptides R+H and D, with or without cholesterol.

### Example 4. Effect of pH During Polymer Coating of Lentiviral Vectors ±VSV-G.

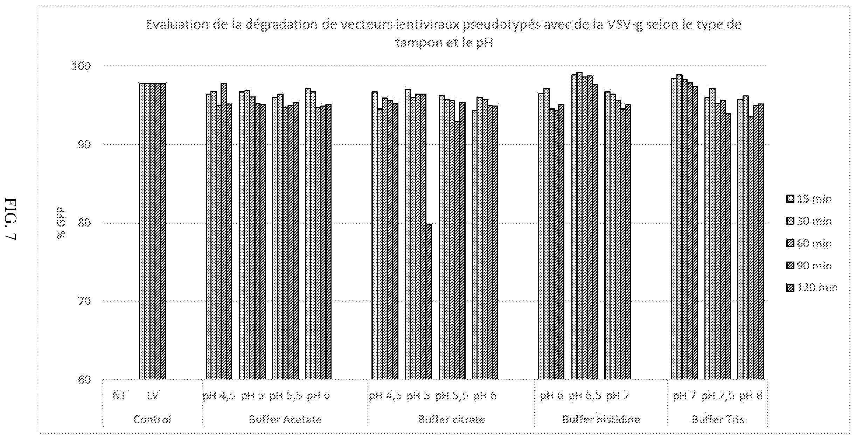

PBAE-coated LV lacking VSV-G or expressing VSV-G were prepared and their transduction of Jurkat cells carried out as described in Example 3, except that the cells were grown in suspension and the pH of the coating solution was varied. Acetate buffer was used at pH between 4.5 and 6, citrate buffer was used at pH between 4.5 and 6, histidine buffer was used at pH between 6 and 7, and Tris buffer was used as pH between 7 and 8. At each pH condition, the number of polymer molecules per LV particle was varied from 10⁶ to 10¹⁰.. The stability of the VSV-G+ LV is shown in Fig. 7. The vectors were stable over the pH range from 4.5 to 8.0 and can be kept at those pH ranges up to 120 mins

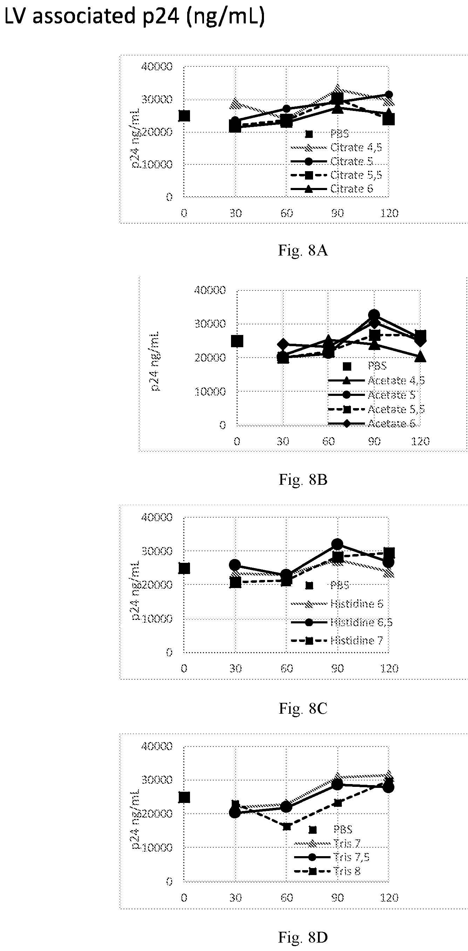

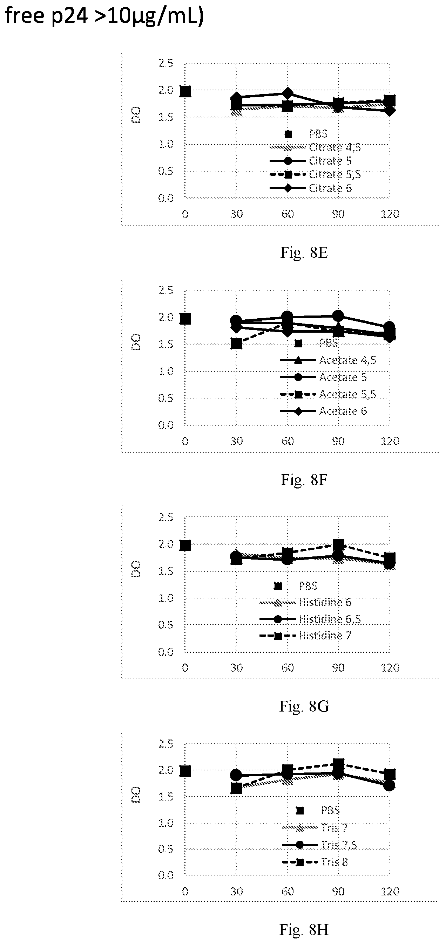

Figs. 8A-8H show the results of an experiment to test the stability of bald LV particles in the different pH buffers without polymer encapsulation. For VSV-G+ LV, integrity was assessed by measuring their ability to transduce HEK293T cells and cause GFP expression (detected by FACS). For VSV-G- (bald) LV, integrity was assessed by measuring release of p24 protein (an indicator of lysis of the vector particles; p24 was measured in supernatants by ELISA), since no transduction was possible using non-polymer-coated bald LV. Physical integrity was determined after 15, 30, 60, 90 and 120 minutes in the different pH buffers. The results indicated no loss of integrity for either type of LV up to 120 min, although the higher pH conditions were slightly less favorable to LV viability than the lower pH conditions. The raw data for bald LV p24 retention and release are shown in Figs. 8A-8H.

Fig. 9 shows the results for encapsulation of bald LV at different pH values, followed by transduction of HEK293T cells and GFP expression detected by FACS. As expected from other experiments discussed in Example 1, complete polymer coating of the bald LV, resulting in transduction-capable LV, required at least about 10⁹ PBAE molecules per LV particle in order to obtain viable particles. A clear pH dependence was observed for the coating process. Higher pH (pH 7) gave practically no infectious LV, whereas coating at pH in the range from about 5.0 to about 6.5 gave good transduction results. The R/H PBAE derivative (PBAE conjugated to CRRR peptides mixed with PBAE-CHHH at a ratio of 60/40 vol/vol, see Example 1) was used for coating in this experiment. Significantly, Fig. 9 also shows that partial encapsulation by polymer results in a lack of transduction capability, which demonstrates that the use of viral vectors lacking envelope protein will enhance the safety profile. Without this feature, vector particles whose polymer coating is lost, partially or completely, or becomes partially degraded, or vector particles that have been incompletely coated during production, could represent a safety concern due to possible transduction of non-targeted cells.

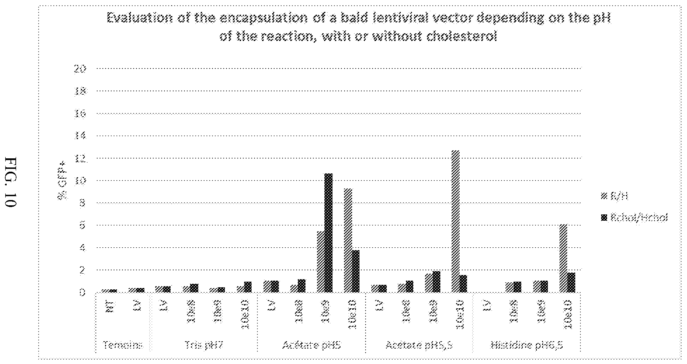

Fig. 10 shows results of an experiment similar to that depicted in FIG. 9, but using a mixture (60/40) of the R-cholesterol-PBAE and H-cholesterol-PBAE polymers. The addition of cholesterol lowered the polymer/LV particle ratio required to fully encapsulate the bald LV at low pH.

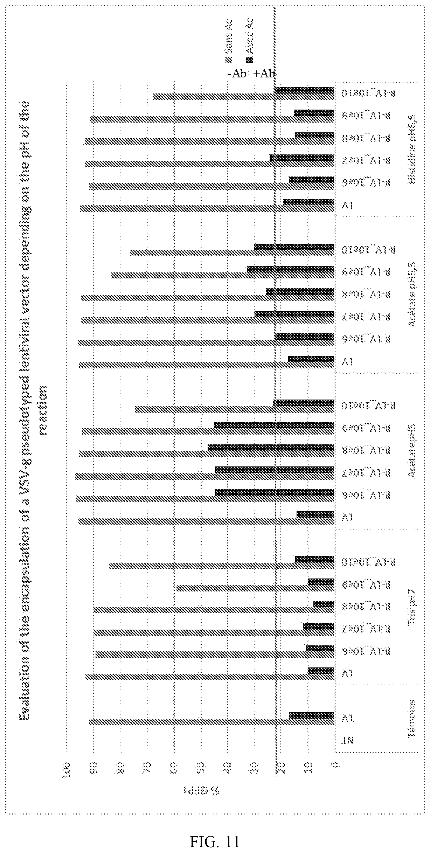

An experiment testing the coating of the VSV-G+ LV at different pH values is shown in Fig. 11. The R-PBAE polymer was used for encapsulation, and the sensitivity to anti-VSV-G+ antibody also was tested. The results indicate little pH sensitivity to overall transduction rates, but antibody sensitivity (indicative of incomplete polymer coating) was greater at pH 6.5 than at lower pH values. Further indication of more compete coating at lower pH is seen as the higher baseline transduction in the presence of anti-VSV-G antibody at pH 5.

### Example 4. Transduction of Lymphocytes Using Encapsulated Lentiviral Vectors ±VSV-G. Transduction of Jurkat Cells and Human Lymphocytes

For the quantification of transduction, Jurkat cells were seeded in 24-well plates at a density of 8×10⁴ cells per well in complete medium containing 10% FBS and 1 % penicillin/streptomycin. 300 µL of encapsulated vector was added to the cells. After 2 h incubation at 37 °C, 5 % CO₂, 500 µL of fresh complete medium was added to each well. The percentage of cells expressing GFP or mCherry transgenes was determined 72 h post-transduction with an Attune NxT flow cytometer (Thermo Fisher) using the BL1 or YL1 channel respectively. The phenotype of transduced cells expressing GFP transgene was determined by flow cytometry staining with different antibodies specific for the following cell types following manufacturer's instructions (BD Biosciences): CD3 (T lymphocytes) and CD19 (B lymphocytes).

The transduction of human lymphocytes was carried out with the same method except that 10⁵ cells/well were seeded.

### Cytotoxicity

Human lymphocytes were seeded in 24-well plates at a density of 5×10⁵ cells per well in complete medium containing 10% FBS and 1 % penicillin/streptomycin. 300 µL of encapsulated CAR CD19 expressing vector was added to the cells. After 2 h incubation, 500 µL of fresh complete medium was added to each well. After 48 h incubation at 37 °C, 5 % CO₂, CD19-positive Ramos tumor cells were added at lymphocyte/tumor cell ratios of 50:1 or 1:1. After 4 h incubation at 37 °C, 5 % CO₂, cells expressing the CAR CD19 were stained with Protein L and detected with an ATTUNE NXT flow cytometer (Thermo Fisher) using the YL1 channel. CD19-positive cells and lymphocytes were quantified by flow cytometry staining with CD19- or CD3-specific antibodies (BD Biosciences) according to manufacturer's instructions.

### Mouse PBMC Preparation and Transduction

Freshly prepared mouse Peripheral Blood Mononuclear Cells (PBMCs) were isolated from heparinized whole blood by using LEUCOSEP tubes (Greiner bio--one) filled with FICOLL-PAQUE PREMIUM 1.084 (GE Healthcare). After blood dilution with DPBS, the PBMCs were separated over a FICOLL density gradient, washed twice with DPBS and resuspended to the desired cell density in culture medium.

Cells were seeded in 24-well plates at a density of 2 × 10⁵ cells per well in RPMI medium containing 10% FBS and 1 % penicillin/streptomycin. 300 µL of encapsulated vector was added to the cells. After 2 h incubation at 37 °C, 5 % CO₂, 500 µL of fresh complete medium was added to each well. The percentage of cells expressing GFP was determined 48 h post-transduction with an Attune NxT flow cytometer using the BL1 channel. The phenotype of transduced cells expressing GFP transgene was determined by flow cytometry staining with different antibodies specific for the following cell types following manufacturer's instructions (BD Biosciences): CD4 (helper T lymphocytes), CD8 (cytotoxic lymphocytes), CD19 (B lymphocytes), Ly-6G (granulocytes), CD11b (macrophages), CD11c (dendritic cells) and F4/80 (monocytes).

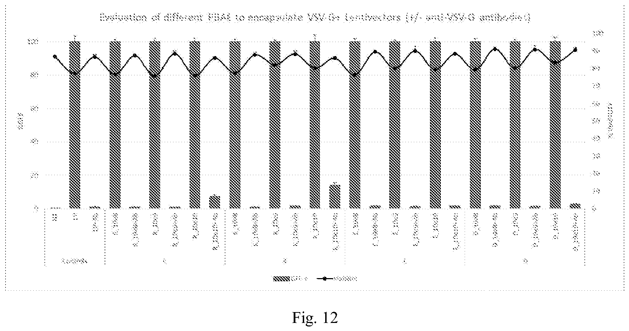

Fig. 12 shows the GFP expression and cell viability of Jurkat cells transduced with the indicated PBAE-encapsulated LV in the presence and absence of anti-VSV-G antibody. The results are normalized to the minus antibody conditions as 100%. A wide variety of encapsulation polymers and polymer/vector ratios were not able to block inhibition by the antibody, indicating that the VSV-G protein protruded through the polymer coating under all conditions probed and mediated transduction.

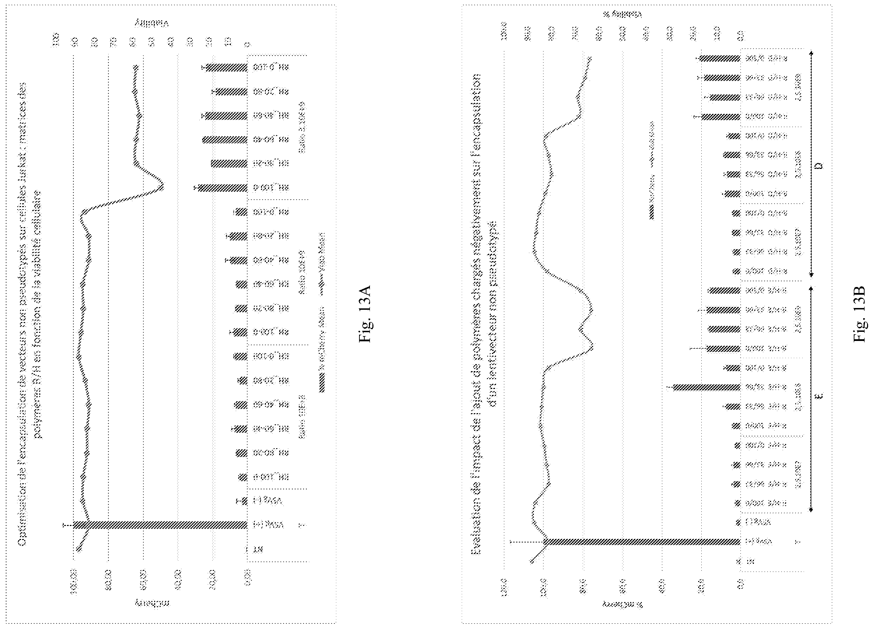

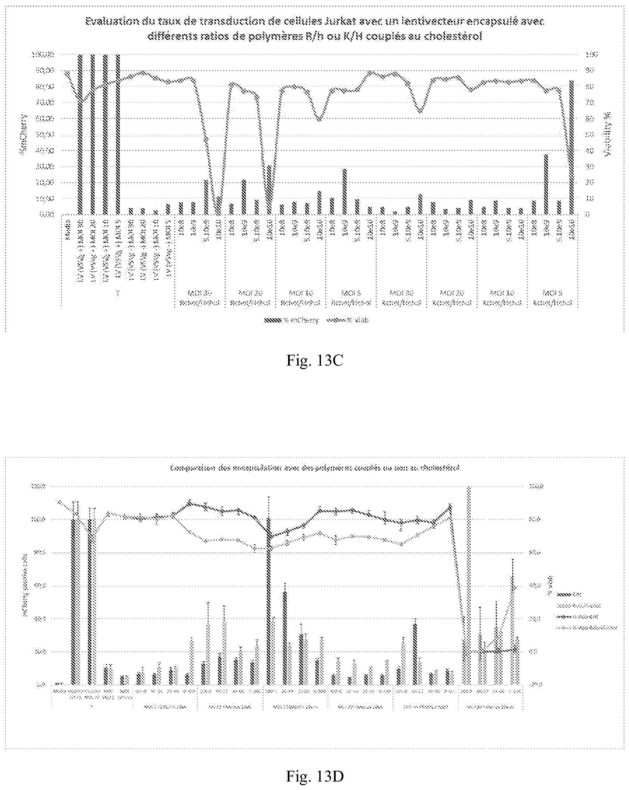

In Figs. 13A-13D, mCherry expression is shown as a measure of transduction of Jurkat cells by bald LV encapsulated by the indicated PBAE polymers, with and without cholesterol conjugation, and at a variety of different polymer/vector ratios and polymer net charge.

The results of transduction of human lymphocytes using different vectors are shown in Figs. 14A-14D. The number of vector particles per lymphocyte increase from left to right in each figure. Fig. 14A shows the results obtained for typical VSV-G⁺ LV; little transduction was observed, as was also the case for the nonencapsulated VSV-G⁻ (bald) LV shown in Fig. 14B. However, the encapsulation of the bald LV with R/H (60/40 vol/vol), ratio 1.10e+9 PBAE per LV with or without 10E+4 PGA-Fab led to successful and dose-dependent transduction as shown in Fig. 14C. In the experiment shown in Fig. 14D, strong transduction also was obtained after adding a targeting moiety (PGA Fab) to the PBAE-encapsulated bald LV. The experiment whose results are shown in Fig. 14E demonstrates that further transduction of human lymphocytes with a CAR specific for CD19 was compatible with a strong transduction of the LV transgene. Further, the results shown in Fig. 14F reveal that the CD19-specific CAR transduced human lymphocytes were capable of cytotoxic action against CD19-positive cancer cells.

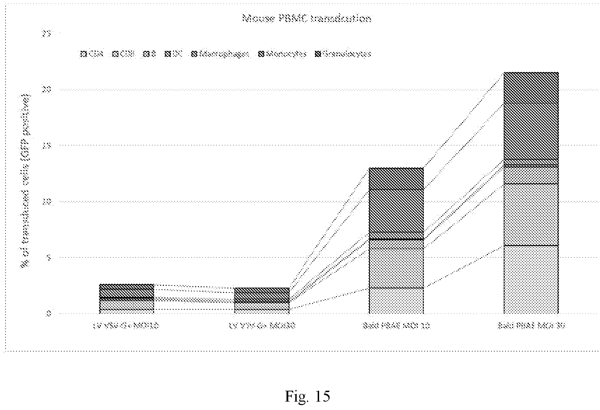

Fig. 15 shows the results of experiments in which mouse PBMC were transfected with either VSV-G⁺ nonencapsulated LV (two bars at left) or PBAE-encapsulated bald LV (two bars at right). The encapsulated bald LV showed much stronger transduction than did the VSV-G⁺ vectors for each cell type present in the PBMC preparation.

References:
Nayerossadat, Maedeh, T, Ali, PA. Viral and nonviral delivery systems for gene delivery, *Adv Biomed Res,* 2102, 1:27
Green, JJ Langer, R, and Anderson, DG, A. Combinatorial Polymer Library Approach Yields Insight into Nonviral Gene Delivery, Acc. Chem. Res, 2008, 41 (6), pp 749-759
He Y, Munn D, Falo LD Jr. Recombinant lentivector as a genetic immunization vehicle for antitumor immunity, Expert Rev Vaccines, 2007 Dec;6(6):913-24
Zennou V, Petit C, Guetard D, Nerhbass U, Montagnier L, Charneau P. HIV-1 genome nuclear import is mediated by a central DNA flap, Cell. 2000 Apr 14;101(2):173-85
Arhel NJ, Souquere-Besse S, Munier S, Souque P, Guadagnini S, Rutherford S, Prévost MC, Allen TD, Charneau P. HIV-1 DNA Flap formation promotes uncoating of the pre-integration complex at the nuclear pore, EMBO J. 2007 Jun 20;26(12):3025-37
Coutant F, Sanchez David RY, Félix T, Boulay A, Caleechurn L, Souque P, Thouvenot C, Bourgouin C, Beignon AS, Charneau P. A nonintegrative lentiviral vector-based vaccine provides long-term sterile protection against malaria, PLoS One. 2012;7(11)
Karwacz K, Mukherjee S, Apolonia L, Blundell MP, Bouma G, Escors D, Collins MK, Thrasher AJ. Nonintegrating lentivector vaccines stimulate prolonged T-cell and antibody responses and are effective in tumor therapy, J Virol. 2009 Apr;83(7):3094-103
Anderson, DJ, Proc. Natl. Acad. Sci. USA 2004, 101:16028-33.
Lynn DM, Langer, R, J. Am Chem Soc 2000, 122:10761-10768.
Mangraviti, et al., ACS Nano 2015, 9:1236-1249.
Negri DR, Michelini Z, Baroncelli S, Spada M, Vendetti S, Buffa V, Bona R, Leone P, Klotman ME, Cara A. Successful immunization with a single injection of non-integrating lentiviral vector, Mol Ther. 2007 Sep;15(9):1716-23
Hu B, Dai B, Wang P. Vaccines delivered by integration-deficient lentiviral vectors targeting dendritic cells induces strong antigen-specific immunity, Vaccine. 2010 Sep 24;28(41):6675-83
Montenarh M. Biochemical properties of the growth suppressor/oncoprotein p53. Oncogene. 1992 Sep;7(9):1673-80

As used herein, "consisting essentially of" allows the inclusion of materials or steps that do not materially affect the basic and novel characteristics of the claim. Any recitation herein of the term "comprising", particularly in a description of components of a composition or in a description of elements of a device, can be exchanged with "consisting essentially of" or "consisting of".

While the present technology has been described in conjunction with certain preferred embodiments, one of ordinary skill, after reading the foregoing specification, will be able to effect various changes, substitutions of equivalents, and other alterations to the compositions and methods set forth herein.

## Claims

1. A nanoparticle comprising a viral vector coated with a polymer or a mixture of polymers, wherein the viral vector comprises a transgene, and wherein the nanoparticle functions as a vehicle for delivering the transgene to eukaryotic cells, wherein the viral vector lacks envelope protein.

2. The nanoparticle of claim 1, wherein the polymer or the mixture of polymers comprises a poly(beta-amino ester) having the formula 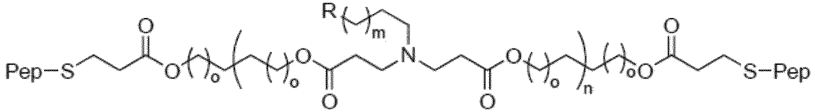 wherein each Pep is an oligopeptide; wherein R is OH, CH₃, or cholesterol; and wherein m ranges from 1 to 20. n ranges from 1 to 100, and o ranges from 1 to 10.

3. The nanoparticle of claim 2, wherein each oligopeptide comprises at least three amino acid residues selected from the group consisting of arginine (R), glutamic acid (E), lysine (K), aspartic acid (D), histidine (H), and cysteine (C), preferably wherein the amino acid sequence of at least one of the oligopeptides is CRRR (SEQ ID NO:1), or CHHH (SEQ ID NO:2), or CKKK (SEQ ID NO:3), or CEEE (SEQ ID NO:4), or CDDD (SEQ ID NO:5).

4. The nanoparticle of any of the preceding claims, wherein the polymer or mixture of polymers has a net positive charge or a net negative charge at pH 7.

5. The nanoparticle of any of the preceding claims, wherein the nanoparticle contains from 10⁸ to 10¹² polymer molecules per vector, preferably wherein the nanoparticle contains from about 10⁹ to about 10¹⁰ polymer molecules per vector.

6. The nanoparticle of any of the preceding claims, wherein the viral vector is a retroviral vector, preferably wherein the retroviral vector is a lentiviral vector.

7. The nanoparticle of any of the preceding claims, further comprising one or more targeting moieties linked to one or more polymer molecules, preferably wherein the targeting moiety is selected from the group consisting of antibodies, antibody fragments, scFvs, antibody-like protein scaffolds, oligopeptides, aptamers, L-RNA aptamers, and ligands for cell surface receptors; for example the targeting moiety is an anti-CD3 antibody or an anti-CD3 aptamer.

8. The nanoparticle of any of the preceding claims, wherein the transgene encodes a chimeric antigen receptor.

9. The nanoparticle of any of the preceding claims that has a zeta potential from about -15 mV to about +15 mV.

10. The nanoparticle of any of the preceding claims that lacks VSV-G envelope protein, preferably wherein the nanoparticle has an improved safety profile for in vivo use compared to a similar nanoparticle comprising VSV-G envelope protein.

11. The nanoparticle of any of the preceding claims, wherein the viral vector lacks viral envelope protein, and wherein the nanoparticle is only capable of transducing a mammalian cell above a threshold number of polymer molecules per vector, preferably::
(i) wherein below said threshold number of polymer molecules per vector, the amount of polymer is insufficient to completely coat the vector, and/or
(ii) wherein below said threshold number of polymer molecules per vector, the nanoparticle is structurally unstable or subject to dissociation of polymer molecules from the nanoparticle, and/or
(iii) wherein the nanoparticle has an improved safety profile for in vivo use compared to a vector or polymer-encapsulated vector that lacks said threshold.

12. A method of making the nanoparticle of any of the preceding claims, the method comprising the steps of:
(a) providing a viral vector comprising a transgene, wherein the viral vector lacks envelope protein;
(b) providing a polymer or a mixture of polymers, and optionally one or more targeting moieties; and
(c) contacting the viral vector and the polymer or the mixture of polymers, and optionally the one or more targeting moieties, whereby the viral vector and the polymer or the mixture of polymers combine to form said nanoparticle.

13. The method of claim 12, wherein:
(i) step (c) is performed at a pH of about 5.0 to about 6.5, preferably at a pH of about 5.5 to 6.0, or step (c) is performed at a pH where the polymer or the mixture of polymers has a net positive charge and the retroviral vector has a negative surface potential, and/or
(ii) the polymer or the mixture of polymers comprises a poly(beta-amino ester) having the formula 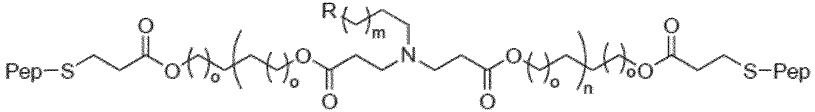 wherein each Pep is an oligopeptide; wherein R is OH, CH₃, or cholesterol; and wherein m ranges from 1 to 20, n ranges from 1 to 100, and o ranges from 1 to 10, preferably wherein each oligopeptide comprises at least three amino acid residues selected from the group consisting of R, E, K, D, H, and C, and/or
(iii) the retroviral vector is a retroviral vector, such as a lentiviral vector, and/or
(iv) the method further comprising contacting the vector with one or more targeting moieties, optionally wherein the targeting moieties are linked to the polymer or mixture of particles.

14. The nanoparticle of any of claims 1-11 for use in a method of treating a disease, such as cancer, the method comprising administering a composition comprising a plurality of said nanoparticles to a subject in need thereof, whereby cells of the subject are transduced by the retroviral vector and the transgene is expressed in the transduced cells.

15. A method of transducing cells *in vitro,* the method comprising contacting cultured cells with a plurality of nanoparticles of any of claims 1-11, whereby at least some of the cultured cells are transduced by the retroviral vector and the transgene is specifically expressed in the transduced cells, preferably wherein the nanoparticles are targeted to CD3-positive cells and the transgene is specifically expressed in CD3-positive cells, or wherein the transgene encodes a chimeric antigen receptor.

16. The nanoparticle of any of claims 1-11 for use in a method of performing gene therapy, the method comprising contacting cells *in vitro* or within a living organism with a plurality of said nanoparticles, whereby the transgene is expressed in the cells.

17. A nanoparticle comprising a viral vector coated with a polymer or a mixture of polymers, wherein the viral vector comprises a reduced level of envelope protein, and comprises a transgene, wherein the nanoparticle functions as a vehicle for delivering the transgene to eukaryotic cells, and wherein the reduced level of envelope protein is insufficient to promote cellular entry of the vector and transduction of the cells by the vector, preferably wherein the reduced level of envelope protein is 5% or less of an amount of envelope protein in a functional virus from which the vector was derived.

## Patentansprüche

1. Nanopartikel, umfassend einen viralen Vektor, der mit einem Polymer oder einem Gemisch von Polymeren beschichtet ist, wobei der virale Vektor ein Transgen umfasst und wobei der Nanopartikel als Vehikel für die Abgabe des Transgens an eukaryontische Zellen fungiert, wobei dem viralen Vektor ein Hüllprotein fehlt.

2. Nanopartikel nach Anspruch 1, wobei das Polymer oder das Polymergemisch einen Poly(beta-aminoester) mit der folgenden Formel umfasst 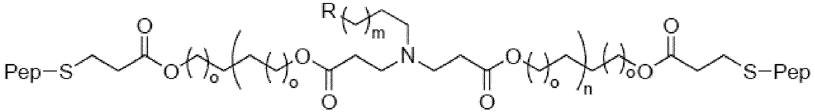 wobei jedes Pep ein Oligopeptid ist, wobei R OH, CH₃ oder Cholesterin ist und wobei m im Bereich von 1 bis 20, n im Bereich von 1 bis 100 und o im Bereich von 1 bis 10 liegt.

3. Nanopartikel nach Anspruch 2, wobei jedes Oligopeptid mindestens drei Aminosäurereste umfasst, die aus der Gruppe ausgewählt sind, die aus Arginin (R), Glutaminsäure (E), Lysin (K), Asparaginsäure (D), Histidin (H) und Cystein (C) besteht, wobei die Aminosäuresequenz von mindestens einem der Oligopeptide vorzugsweise CRRR (SEQ ID NO:1), oder CHHH (SEQ ID NO:2), oder CKKK (SEQ ID NO:3), oder CEEE (SEQ ID NO:4), oder CDDD (SEQ ID NO:5).

4. Nanopartikel nach einem der vorhergehenden Ansprüche, wobei das Polymer oder die Polymermischung bei pH 7 eine positive Nettoladung oder eine negative Nettoladung aufweist.

5. Nanopartikel nach einem der vorhergehenden Ansprüche, wobei der Nanopartikel 10⁸ bis 10¹² Polymermoleküle pro Vektor enthält, wobei der Nanopartikel vorzugsweise etwa 10⁹ bis etwa 10¹⁰ Polymermoleküle pro Vektor enthält.

6. Nanopartikel nach einem der vorhergehenden Ansprüche, wobei der virale Vektor ein retroviraler Vektor ist, wobei der retrovirale Vektor vorzugsweise ein lentiviraler Vektor ist.

7. Nanopartikel nach einem der vorhergehenden Ansprüche, ferner umfassend eine oder mehrere Targeting-Einheiten, die an ein oder mehrere Polymermoleküle gebunden sind, wobei die Targeting-Einheit vorzugsweise aus der Gruppe ausgewählt ist, die aus Antikörpern, Antikörperfragmenten, scFvs, antikörperähnlichen Proteingerüsten, Oligopeptiden, Aptameren, L-RNA-Aptameren und Liganden für Zelloberflächenrezeptoren besteht; die Targeting-Einheit ist beispielsweise ein Anti-CD3-Antikörper oder ein Anti-CD3-Aptamer.

8. Nanopartikel nach einem der vorhergehenden Ansprüche, wobei das Transgen für einen chimären Antigenrezeptor kodiert.

9. Nanopartikel nach einem der vorhergehenden Ansprüche, das ein Zetapotenzial von etwa -15 mV bis etwa +15 mV aufweist.

10. Nanopartikel nach einem der vorhergehenden Ansprüche, dem das VSV-G-Hüllprotein fehlt, wobei das Nanopartikel vorzugsweise ein verbessertes Sicherheitsprofil für die In-vivo-Verwendung im Vergleich zu einem ähnlichen Nanopartikel mit VSV-G-Hüllprotein aufweist.

11. Nanopartikel nach einem der vorhergehenden Ansprüche, wobei dem viralen Vektor virales Hüllprotein fehlt und wobei das Nanopartikel nur oberhalb einer Schwellenwertzahl von Polymermolekülen pro Vektor in der Lage ist, eine Säugerzelle zu transduzieren, vorzugsweise:
(i) wobei unterhalb der Schwellenwertanzahl von Polymermolekülen pro Vektor die Menge an Polymer nicht ausreicht, um den Vektor vollständig zu umhüllen, und/oder
(ii) wobei unterhalb der Schwellenwertanzahl von Polymermolekülen pro Vektor das Nanopartikel strukturell instabil ist oder der Dissoziation von Polymermolekülen von dem Nanopartikel unterliegt, und/oder
(iii) wobei das Nanopartikel ein verbessertes Sicherheitsprofil für die In-vivo-Verwendung im Vergleich zu einem Vektor oder polymerverkapselten Vektor aufweist, dem dieser Schwellenwert fehlt.

12. Verfahren zur Herstellung eines Nanopartikels nach einem der vorhergehenden Ansprüche, wobei das Verfahren die folgenden Schritte umfasst:
(a) Bereitstellen eines viralen Vektors, der ein Transgen enthält, wobei dem viralen Vektor ein Hüllprotein fehlt;
(b) Bereitstellen eines Polymers oder einer Mischung von Polymeren und gegebenenfalls einer oder mehrerer Zielgruppeneinheiten und
(c) In-Kontakt-Bringen des viralen Vektors und des Polymers oder des Polymergemischs und gegebenenfalls des einen oder der mehreren Targeting-Anteile, wodurch sich der virale Vektor und das Polymer oder das Polymergemisch zur Bildung des Nanopartikels verbinden.

13. Verfahren nach Anspruch 12, wobei:
(i) Schritt (c) bei einem pH-Wert von etwa 5,0 bis etwa 6,5, vorzugsweise bei einem pH-Wert von etwa 5,5 bis 6,0, durchgeführt wird, oder Schritt (c) bei einem pH-Wert durchgeführt wird, bei dem das Polymer oder das Gemisch von Polymeren eine positive Nettoladung aufweist und der retrovirale Vektor ein negatives Oberflächenpotential hat, und/oder
(ii) das Polymer oder die Polymermischung einen Poly(beta-aminoester) mit der Formel 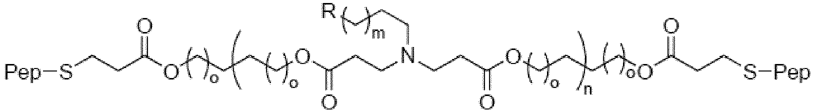 umfasst, wobei jedes Pep ein Oligopeptid ist, wobei R OH, CH₃ oder Cholesterin ist und wobei m im Bereich von 1 bis 20, n im Bereich von 1 bis 100 und o im Bereich von 1 bis 10 liegt, wobei vorzugsweise jedes Oligopeptid mindestens drei Aminosäurereste umfasst, die aus der Gruppe ausgewählt sind, die aus R, E, K, D, H und C besteht, und/oder
(iii) der retrovirale Vektor ein retroviraler Vektor, wie z.B. ein lentiviraler Vektor, ist und/oder
(iv) das Verfahren ferner das In-Kontakt-Bringen des Vektors mit einer oder mehreren zielgerichteten Einheiten umfasst, wobei die zielgerichteten Einheiten gegebenenfalls mit dem Polymer oder dem Partikelgemisch verbunden sind.

14. Nanopartikel nach einem der Ansprüche 1 bis 11 zur Verwendung in einem Verfahren zur Behandlung einer Krankheit, wie z.B. Krebs, wobei das Verfahren die Verabreichung einer Zusammensetzung, die eine Vielzahl der Nanopartikel umfasst, an ein Subjekt, das diese benötigt, umfasst, wodurch Zellen des Subjekts durch den retroviralen Vektor transduziert werden und das Transgen in den transduzierten Zellen exprimiert wird.

15. Verfahren zur Transduktion von Zellen *in vitro,* wobei das Verfahren das In-Kontakt-Bringen von kultivierten Zellen mit einer Vielzahl von Nanopartikeln nach einem der Ansprüche 1 bis 11 umfasst, wodurch mindestens einige der kultivierten Zellen durch den retroviralen Vektor transduziert werden und das Transgen spezifisch in den transduzierten Zellen exprimiert wird, wobei die Nanopartikel vorzugsweise auf CD3-positive Zellen abzielen und das Transgen spezifisch in CD3-positiven Zellen exprimiert wird, oder wobei das Transgen für einen chimären Antigenrezeptor kodiert.

16. Nanopartikel nach einem der Ansprüche 1 bis 11 zur Verwendung in einem Verfahren zur Durchführung einer Gentherapie, wobei das Verfahren das In-Kontakt-Bringen von Zellen *in vitro* oder innerhalb eines lebenden Organismus mit einer Vielzahl der Nanopartikel umfasst, wodurch das Transgen in den Zellen exprimiert wird.

17. Nanopartikel, umfassend einen viralen Vektor, der mit einem Polymer oder einem Gemisch von Polymeren beschichtet ist, wobei der virale Vektor einen reduzierten Gehalt an Hüllprotein und ein Transgen umfasst, wobei das Nanopartikel als Vehikel für die Abgabe des Transgens an eukaryontische Zellen fungiert, und wobei der reduzierte Gehalt an Hüllprotein nicht ausreicht, um den zellulären Eintritt des Vektors und die Transduktion der Zellen durch den Vektor zu fördern, wobei der reduzierte Gehalt an Hüllprotein vorzugsweise 5 % oder weniger der Menge an Hüllprotein in einem funktionalen Virus beträgt, von dem der Vektor abgeleitet wurde.

## Revendications

1. Nanoparticule comprenant un vecteur viral revêtu d'un polymère ou d'un mélange de polymères, dans laquelle le vecteur viral comprend un transgène, et dans laquelle la nanoparticule fonctionne comme un véhicule pour délivrer le transgène à des cellules eucaryotes, dans laquelle le vecteur viral manque de protéine d'enveloppe.

2. Nanoparticule selon la revendication 1, dans laquelle le polymère ou le mélange de polymères comprend un poly(bêta-amino ester) ayant la formule 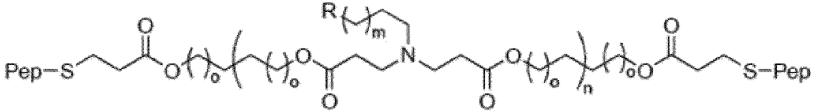 dans laquelle chaque Pep est un oligopeptide ; dans laquelle R est OH, CH₃, ou le cholestérol ; et dans laquelle m est de 1 à 20, n est de 1 à 100, et o est de 1 à 10.

3. Nanoparticule selon la revendication 2, dans laquelle chaque oligopeptide comprend au moins trois résidus d'acide aminé choisis dans le groupe consistant en arginine (R), acide glutamique (E), lysine (K), acide aspartique (D), histidine (H), et cystéine (C), de préférence dans laquelle la séquence d'acide aminé d'au moins un des oligopeptides est CRRR (SEQ ID NO:1), ou CHHH (SEQ ID NO:2), ou CKKK (SEQ ID NO:3), ou CEEE (SEQ ID NO:4), ou CDDD (SEQ ID NO:5).

4. Nanoparticule selon l'une quelconque des revendications précédentes, dans laquelle le polymère ou mélange de polymères présente une charge positive nette ou une charge négative nette à pH 7.

5. Nanoparticule selon l'une quelconque des revendications précédentes, dans laquelle la nanoparticule contient de 10⁸ à 10¹² molécules de polymère par vecteur, de préférence dans laquelle la nanoparticule contient d'environ 10⁹ à environ 10¹⁰ molécules de polymère par vecteur.

6. Nanoparticule selon l'une quelconque des revendications précédentes, dans laquelle le vecteur viral est un vecteur rétroviral, de préférence dans laquelle le vecteur rétroviral est un vecteur lentiviral.

7. Nanoparticule selon l'une quelconque des revendications précédentes, comprenant de plus une ou plusieurs moitiés cible liées à une ou plusieurs molécules de polymère, de préférence dans laquelle la moitié cible est choisie dans le groupe consistant en anticorps, fragments d'anticorps, scFvs, matrices protéiques semblables à un anticorps, oligopeptides, aptamères, apatamères L-ARN , et ligands pour récepteurs de surface cellulaire ; par exemple la moitié cible est un anticorps anti-CD3 ou un aptamère anti-CD3 .

8. Nanoparticule selon l'une quelconque des revendications précédentes, dans laquelle le transgène code un récepteur d'antigène chimérique.

9. Nanoparticule selon l'une quelconque des revendications précédentes qui présente un potentiel zêta d'environ -15 mV à environ +15 mV.

10. Nanoparticule selon l'une quelconque des revendications précédentes qui manquent de protéine d'enveloppe VSV-G, de préférence dans laquelle la nanoparticule présente un profil de sécurité amélioré pour une utilisation in vivo en comparaison à une nanoparticule similaire comprenant une protéine d'enveloppe VSV-G.

11. Nanoparticule selon l'une quelconque des revendications précédentes, dans laquelle le vecteur viral manque de protéine d'enveloppe virale, et dans laquelle la nanoparticule est seulement capable de transduction d'une cellule mammifère au-dessus d'un nombre seuil de molécules de polymère par vecteur, de préférence :
(i) dans laquelle sous ledit nombre seuil de molécules de polymère par vecteur, la quantité de polymère est insuffisante pour revêtir complètement le vecteur, et/ou
(ii) dans laquelle sous ledit nombre seuil de molécules de polymère par vecteur, la nanoparticule est structurellement instable ou soumise à une dissociation de molécules de polymère à partir de la nanoparticule, et/ou
(iii) dans laquelle la nanoparticule présente un profil de sûreté amélioré pour une utilisation in vivo en comparaison à un vecteur ou vecteur encapsulé par un polymère qui manque dudit seuil.

12. Procédé de fabrication de la nanoparticule selon l'une quelconque des revendications précédentes, le procédé comprenant les étapes de :
(a) fourniture d'un vecteur viral comprenant un transgène, dans lequel le vecteur viral manque de protéine d'enveloppe ;
(b) fourniture d'un polymère ou d'un mélange de polymères, et éventuellement d'une ou plusieurs moitiés cible ; et
(c) mise en contact du vecteur viral et du polymère ou du mélange de polymères, et éventuellement de la une ou plusieurs moitiés cible, le vecteur viral et le polymère ou le mélange de polymères se combinent par-là pour former ladite nanoparticule.

13. Procédé selon la revendication 12, dans lequel :
(i) l'étape (c) est réalisée à un pH d'environ 5,0 à environ 6,5, de préférence à un pH d'environ 5,5 à 6,0, ou l'étape (c) est réalisée à un pH où le polymère ou le mélange de polymères présente une charge positive nette et le vecteur rétroviral présente un potentiel de surface négatif, et/ou
(ii) le polymère ou le mélange de polymères comprend un poly(bêta-amino ester) ayant la formule 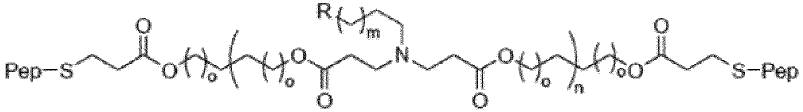 dans laquelle chaque PEP est un oligopeptide ; dans laquelle R est OH, CH₃, ou le cholestérol ; et dans laquelle m est de 1 à 20, n est de 1 à 100 et o est de 1 à 10, de préférence dans laquelle chaque oligopeptide comprend au moins trois résidus d'acide aminé choisis dans le groupe consistant en R, E, K, D, H, et C, et/ou
(iii) le vecteur rétroviral est un vecteur rétroviral, tel qu'un vecteur lentiviral, et/ou
(iv) le procédé comprenant de plus la mise en contact du vecteur avec une ou plusieurs moitiés cible, éventuellement dans lequel les moitiés cible sont liées au polymère ou mélange de particules.

14. Nanoparticule selon l'une quelconque des revendications 1-11 pour une utilisation dans un procédé de traitement d'une maladie, telle que le cancer, le procédé comprenant l'administration d'une composition comprenant plusieurs desdites nanoparticules à un sujet en besoin de celle-ci, sur quoi des cellules du sujet sont soumises à une transduction par le vecteur rétroviral et le transgène est exprimé dans les cellules transduites.

15. Procédé de transduction de cellules *in vitro,* le procédé comprenant la mise en contact de cellules mises en culture avec plusieurs nanoparticules selon l'une quelconque des revendications 1-11, sur quoi au moins certaines des cellules mises en culture sont transduites par le vecteur rétroviral et le transgène est spécifiquement exprimé dans les cellules transduites, de préférence dans lequel les nanoparticules sont ciblées par rapport aux cellules CD3-positives et le transgène est spécifiquement exprimé dans des cellules CD3-positives, ou dans lequel le transgène code un récepteur antigène chimérique.

16. Nanoparticule selon l'une quelconque des revendications 1-11 pour une utilisation dans un procédé de réalisation d'une thérapie génique, le procédé comprenant la mise en contact de cellules *in vitro* ou à l'intérieur d'un organisme vivant avec plusieurs desdites nanoparticules, sur quoi le transgène est exprimé dans les cellules.

17. Nanoparticule comprenant un vecteur viral revêtu d'un polymère ou d'un mélange de polymères, dans laquelle le vecteur viral comprend un niveau réduit de protéine d'enveloppe, et comprend un transgène, dans laquelle la nanoparticule fonctionne comme un véhicule pour délivrer le transgène à des cellules eucaryotes, et dans laquelle le niveau réduit de protéine d'enveloppe est insuffisant pour promouvoir une entrée cellulaire du vecteur et une transduction des cellules par le vecteur, de préférence dans laquelle le niveau réduit de la protéine d'enveloppe est de 5 % ou inférieur d'une quantité de protéine d'enveloppe dans un virus fonctionnel à partir duquel le vecteur a été dérivé.
